(19) Europäisches Patentamt
European Patent Office
Office européen des brevets

(11) **EP 4 339 883 B1**

(12) **EUROPEAN PATENT SPECIFICATION**

(45) Date of publication and mention
of the grant of the patent:
**29.10.2025   Bulletin 2025/44**

(21) Application number: **22196031.3**

(22) Date of filing: **16.09.2022**

(51) International Patent Classification (IPC):
*G06T 7/00* (2017.01)     *G06T 7/10* (2017.01)
*G06N 3/09* (2023.01)     *G06N 3/08* (2023.01)
*G06N 3/044* (2023.01)     *G06N 3/045* (2023.01)
*G06N 3/0464* (2023.01)     *G06N 3/091* (2023.01)
*G06T 7/11* (2017.01)     *G16H 30/20* (2018.01)
*G16H 30/40* (2018.01)     *G16H 50/20* (2018.01)
*G16H 50/70* (2018.01)

(52) Cooperative Patent Classification (CPC):
**G06N 3/091; G06N 3/0464; G06N 3/09;
G06T 7/0012; G06T 7/11; G16H 30/20;
G16H 30/40; G16H 50/20; G16H 50/70;**
G06T 2207/20081; G06T 2207/20084;
G06T 2207/20104; G06T 2207/30061; G16H 15/00

(54) **TECHNIQUE FOR INTERACTIVE MEDICAL IMAGE SEGMENTATION**

TECHNIK ZUR INTERAKTIVEN SEGMENTIERUNG MEDIZINISCHER BILDER

TECHNIQUE DE SEGMENTATION INTERACTIVE D'IMAGES MEDICALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**20.03.2024   Bulletin 2024/12**

(73) Proprietor: **Siemens Healthineers AG
91301 Forchheim (DE)**

(72) Inventors:
• **MANSOOR, Awais
Potomac, 20854 (US)**

• **GEORGESCU, Bogdan
Princeton, 08540 (US)**
• **GRBIC, Sasa
Plainsboro, 08536 (US)**

(74) Representative: **Schwarz, Claudia
Schwarz + Kollegen
Patentanwälte
Heilmannstraße 19
81479 München (DE)**

(56) References cited:
**EP-B1- 3 639 240       JP-A- 2022 535 219
US-A1- 2020 085 382**

**Description**

TECHNICAL FIELD

A technique for training a neural network for interactive medical image segmentation is provided. In particular, a method, a neural network system, a computer program product and a computer-readable medium are provided.

BACKGROUND ART

Deep-learning (DL) with convolutional neural networks (CNNs) has achieved state-of-the-art performance for automated medical image segmentation. However, automatic segmentation techniques have not demonstrated sufficient accuracy and robustness in results for across-the-board clinical use due to inherent challenges of medical images, such as poor image quality, pathologies, different imaging and segmentation protocols, and variations among patients. Alternatively, interactive segmentation methods (such as, e.g., ITK-Snap, and/or the picture archiving and communication system, PACS, tool) are widely adopted, as integrating the user's knowledge can take into account the application requirements and make it easier to distinguish different tissues. As such, interactive segmentation remains the state-of-the-art for conventional surgical planning and navigation products. Though leveraging user interactions often leads to more robust segmentations, most automated segmentation tools do not offer an efficient mechanism to interact, and most of the time force the user to accept or reject the segmentation in entirety when small manual interactions could in principle make the segmentation acceptable for clinical use.

The document US 2020/085382 A1 describes automated lesion detection, segmentation and longitudinal identification based on CT and MRI.

The document JP 2022/535219 A1 describes an image segmentation method, in which a first segmentation result of a target image is obtained with an assigned probability per category of each pixel. A correction with an assigned category of the correction point is received, based on which a second segmentation result is obtained.

The document EP 3639240 B1 describes a variety of methods for segmenting an image. User interactions in the form of clicks or scribbles are combined with a 1st machine learning system that automatically generates an initial segmentation, which is then refined by a 2nd machine learning system, which takes as input the initial segmentation and any user interactions. Geodesic distance maps are used for the user interactions.

A good interactive method requires as little user time as possible to reduce the burden on users.

There are very few studies on using CNNs for interactive medical imaging segmentation [1]-[3]. This is mainly due to the requirement of large amounts of annotated images for training, the lack of image-specific adaptation and the demanding balance of model complexity, time and memory space efficiency.

A first challenge of using CNNs for interactive segmentation is that conventional CNNs do not generalize well to previously unseen object classes, as they require labeled instances of each object class to be present in the training dataset. For medical images, annotations are often expensive to acquire, as both expertise and time are needed to produce accurate annotations. The lack of training datasets in a training phase limits the performance of CNNs to segment objects for which annotations are not available in an inference phase. The lack of training datasets is especially true for pathological tissues and organs, which also happen to be the ones where conventional automated segmentation techniques fail generally.

Secondly, interactive segmentation often requires image-specific learning to deal with large context variations among different images. However, conventional CNNs are not adaptive to different test images, as parameters of the model are learned from training images and then fixed during the testing, without image-specific adaptation.

Thirdly, fast inference and memory efficiency are demanded for interactive methods. The required speed and memory efficiency may be achieved for two-dimensional (2D) segmentations, but are problematic for three-dimensional (3D) volumes. For example, DeepMedic [4] works on local patches to reduce memory requirements, but results in a slow inference. HighRes3DNet [5] works on an entire volume with relatively fast inference but needs a large amount of graphics processing unit (GPU) memory, leading to high hardware requirements.

To make a CNN-based interactive segmentation method efficient to use, enabling CNNs to respond quickly to user interactions and to work on a machine with limited GPU resources (e.g., a standard desktop PC or a laptop) is desirable. DeepIGeoS [3] combines CNNs with user interactions and has demonstrated good interactivity. However, DeepIGeoS has a lack of adaptability to unseen image contexts and pathologies.

It is therefore an object of the present invention to provide a solution for combining neural networks (NNs), in particular CNNs, with user interactions for medical image segmentation to achieve higher segmentation accuracy and robustness with fewer user interactions and less user time. Alternatively or in addition, an object of the present invention is to achieve segmentations of medical images at high speeds with high accuracy and/or limited memory space. Further alternatively or in addition, it is an object of the present invention to provide a technique for segmenting anatomical structures, in particular pathologies, for which training datasets are scarce.

SUMMARY

This object is solved by a computer-implemented method for training a neural network (NN) for interactive medical image segmentation, by a NN system, by a computer program product, and by a computer-readable storage medium according to the appended independent claims. Advantageous aspects, features and embodiments are described in the dependent claims and in the following description together with advantages.

In the following the solution according to the invention is described with respect to the claimed computer-implemented method as well as with respect to the claimed NN system. Features, advantages or alternative embodiments herein can be assigned to the other claimed objects (e.g., the computer program or a computer program product) and vice versa. In other words, claims for the NN system can be improved with features described or claimed in the context of the method. In this case, the functional features of the method are embodied by structural units of the system and vice versa, respectively.

As to a method aspect, a computer-implemented method for training a neural network (NN) for interactive medical image segmentation is provided. A training phase of the NN comprises a step of receiving, at an input layer of the NN, a first medical image dataset from a training database of medical image datasets as input to the NN. The first medical image dataset comprises a medical image and optionally an annotated segmentation (which may alternatively be denoted as ground truth segmentation).

The training phase of the NN further comprises a step of performing, by one or more hidden layers of the NN with interconnections, a first segmentation of anatomic structures comprised in the medical image of the received first medical image dataset. The training phase of the NN further comprises a step of outputting, at an output layer of the NN, the performed first segmentation for corrections by a user. The training phase of the NN further comprises a step of receiving, at a user interface (UI) at least one correction by the user on the output first segmentation. The training phase of the NN further comprises a step of determining, based on the output first segmentation, a second segmentation taking into account the at least one received correction to the first segmentation.

The training phase of the NN further comprises a step of determining a distance metric between the determined second segmentation and the annotated segmentation (alternatively denoted as ground truth segmentation) comprised in the received medical image dataset in association with the medical image. Alternatively or in addition, the step of determining the distance metric comprises determining a distance metric between the determined second segmentation and the output first segmentation.

The training phase of the NN further comprises a step of combining the determined distance metric, the determined second segmentation and the medical image into a second medical image dataset. The training phase of the NN further comprises a step of receiving, at the input layer of the NN, the combined second medical image dataset as input to the NN. The training phase of the NN further comprises a step of performing, by the one or more hidden layers of the NN with interconnections, a third segmentation of the anatomic structures comprised in the medical image of the received second medical image dataset. Performing the third segmentation comprises changing one or more weights of the interconnections of the NN. The changing of the one or more weights of the interconnections of the NN is based on the determined distance metric comprised in the second medical image dataset.

The training phase of the NN further comprises a step of outputting, at the output layer of the NN, the performed third segmentation.

The training phase of the NN further comprises a step of determining a further distance metric between the output third segmentation, and/or a fourth segmentation based on the third segmentation (e.g., if at least one further correction by the user is received at the UI), and the annotated segmentation (also denoted as ground truth segmentation) comprised in the received second medical image dataset. Alternatively or in addition, the step of determining the further distance metric comprises determining a further distance metric between the output third segmentation and the determined second segmentation, and/or a further distance metric between the (e.g., determined, in particular responsive to one or more further corrections by the user) fourth segmentation and the output third segmentation.

The training phase of the NN still further may comprise a step of selectively repeating at least the steps of receiving a combined further medical image data set, performing a further segmentation of the anatomic structures comprised in the medical image of the received further medical image data set, outputting the performed further segmentation, and determining a (e.g., still) further distance metric between the output further segmentation and the annotated segmentation. Alternatively or in addition, the (e.g., still) further distance metric is determined between the output further segmentation and the directly preceding segmentation.

The selectivity of the step of selectively repeating may alternatively or in addition comprise determining to repeat the steps if the further distance metric, or any still further distance metric, exceeds a predetermined threshold. Alternatively or in addition, the selectivity of the step of selectively repeating comprises determining to not repeat the steps if the further distance metric, or any still further distance metric, falls below the predetermined threshold.

The computer-implemented method may comprise deep learning (DL).

The training database of medical images may comprise annotated medical images. The annotated medical images may also be denoted as labelled images. The annotations and/or labels may comprise segmentations performed by one or more expert(s), in particular a medical practitioner and/or radiologist.

The annotated segmentation may correspond to, or may comprise, a segmentation performed by a medical practitioner, e.g., a radiologist. Alternatively or in addition, the annotated segmentation may correspond to, or may comprise, an annotation and/or label of the first medical image dataset.

The annotated segmentation may also be denoted as ground truth segmentation (briefly: ground truth).

The annotated segmentation may comprise two or more components of, e.g., an anatomical structure and/or a pathological structure (also denoted as pathology or as health-related structure), e.g., the left lung and the right lung, and/or different types of organs, e.g., liver and one or two kidneys.

The step of receiving the first medical image dataset, and/or any further medical image dataset, at the input layer of the NN may comprise extracting the medical image for performing the first segmentation, and/or for performing any further segmentation. Alternatively or in addition, the step of receiving the second medical image dataset (and/or any further medical image dataset) at the input layer of the NN may comprise extracting the distance metric (and/or any further distance metric) and/or the second segmentation (and/or any segmentation comprised in the further medical image dataset) for modifying the respective segmentation, at least within one or more regions of interest (ROI) indicated by distance metric.

The second segmentation may directly and/or indirectly comprise the one or more corrections input by the user. E.g., a change in a contour starting and ending on a contour of the first segmentation may directly provide the second segmentation. Alternatively or in addition, e.g., if a correction by the user is only a line (in particular without endpoints at a contour of the first segmentation), the second segmentation may require a computational effort. E.g., the second segmentation may comprise an output of the NN, or a further NN, based on the first segmentation and the correction by the user as input. Further alternatively or in addition, a direct change of the segmentation, e.g., a change of a contour at one anatomical position, may be combined with an indirect change of the segmentation, e.g., a change of a contour at another anatomical position determined by a NN based on one or more user corrections.

The distance metric may be determined (e.g., computed) using the second segmentation with the one or more corrections by the user taken into account directly and/or indirectly.

The step of "combining" may be interpreted in the sense of automatically generating a further medical image dataset (briefly also: image dataset), based on the distance metric, the determined second segmentation and the medical image of the first medical image dataset. The generated further image dataset may be based on the medical image of the first medical image dataset and may comprise the distance metric and/or the determined second segmentation.

The training database may stem from, or may make use of, a picture archiving and communication system (PACS). PACS may comprise a medical imaging technology which provides economical storage and convenient access to images from multiple imaging modalities (also denoted as: source machine types). Electronic images and reports may be transmitted digitally via PACS, eliminating the need to manually file, retrieve, and/or transport film jackets, e.g., the folders used to store and protect the medical image, e.g., X-ray film.

The universal format for PACS image storage and transfer may be Digital Imaging and Communications in Medicine (DICOM). Combined with available and emerging web technology, PACS may have the ability to deliver timely and efficient access to medical images, interpretations, and related data, in particular comprising annotated segmentations.

The training database of medical images may comprise medical images received from one or more medical imaging devices using at least one medical imaging modality. Alternatively or in addition, the training steps of the NN may be performed using medical images from at least two different medical imaging modalities. The medical images may be received from at least two different medical imaging devices using different medical imaging modalities (e.g., one medical imaging modality per medical imaging device).

By using medical images from at least two different medical imaging modalities, the NN may be trained for a wider variety of images in the inference phase (e.g., as compared to training using a single medical imaging modality).

The inference phase may also be denoted as application phase.

Medical imaging modalities may comprise magnetic resonance imaging (MRI), computed tomography (CT), positron emission tomography (PET), ultrasound (US), and/or radiology (also denoted as X-ray imaging).

The NN may comprise a convolutional neural network (CNN; also: ConvNet).

The NN, in particular the CNN, may comprise a U-net. The U-net may comprise a contracting path and an expansive path, giving it a U-shaped architecture. The contracting path of the U-net may comprise a, e.g., conventional and/or fully, CNN, that comprises a repeated application of convolutions, each followed by a rectified linear unit (ReLU) and a max pooling operation. During the contraction, the spatial information may be reduced while feature information may be increased. The expansive path of the U-net may combine the feature information and spatial information through a sequence of up-convolutions and concatenations with high-resolution features from the contracting path.

Max pooling may comprise a pooling operation that calculates the maximum value for patches of a feature map, and uses it to create a downsampled (and/or pooled) feature map. Max pooling may, e.g., be used after a convolutional layer. Max pooling may add a small amount of translation invariance, e.g., meaning translating the image by a small amount does not significantly affect the values of most pooled outputs.

Alternatively or in addition, the NN may comprise a ResNet (e.g., short for residual neural network) encoder. The ResNet may comprise a CNN architecture, made up of series of residual blocks (ResBlocks) with skip connections, e.g., with the skip connections differentiating ResNets from other CNNs. The skip connections (also denoted as shortcuts) may be used to jump over one or more, in particular, hidden, layers (e.g., HighwayNets may also learn the skip weights themselves through an additional weight matrix for their gates). E.g., the ResNet may be implemented with double-layer skips and/or triple-layer skips that comprise nonlinearities (e.g., accounted for, and/or compensated by, a ReLU) and/or batch normalization in between.

Alternatively or in addition, the CNN may comprise a region based CNN (R-CNN), in particular a Mask R-CNN, and/or one or more transformers.

A mask R-CNN may comprise a (e.g., single) NN tasked to perform object localization and segmentation simultaneously. The Mask R-CNN may perform a step of extracting of one or more regions (also denoted as boxes) from a (e.g., given) image using selective search. The Mask R-CNN may then perform a check if any of the regions (and/or boxes) contains an object and output a binary mask for each region (and/or box).

Further alternatively or in addition, the NN may comprise a DeepMedic. DeepMedic may comprise an efficient multi-scale three-dimensional CNN for segmentation of medical images.

Still further alternatively or in addition, the NN may comprise a HighRes3DNet.

A HighRes3Dnet may be considered as a three counterpart to ResNet. The HighRes3Dnet may comprise a three-dimensional (3D) CNN architecture, made up of series of residual blocks (ResBlocks) with skip connections.

Any one of the segmentations may be performed using an extension of the conventional segmentation tool ITK-SNAP. ITK-SNAP may comprise an interactive software application that allows users to navigate three-dimensional medical images, manually delineate anatomical regions of interest, and/or perform automatic image segmentation.

The one or more corrections by the user (also denoted as: input by the user; briefly: user input) may comprise adding and/or deleting (also denoted as subtracting), e.g., a line and/ or a contour, of a segmentation. Alternatively or in addition, the input by the user may comprise selecting, and/or using, an editing tool. The editing tool may be configured for adding and/or subtracting at least part of the first segmentation.

The one or more corrections by the user may be localized within the medical image. E.g., a correction may correspond to, or may be comprised in, a region of interest (ROI) of the medical image.

The one or more corrections by the user may comprise manual corrections (also denoted as: real corrections) performed by a medical practitioner, e.g., a radiologist. The manual corrections used in the training phase may, e.g., comprise a subset of the annotations comprised in the first medical image dataset.

Alternatively or in addition, the one or more corrections may comprise synthetic corrections and/or simulated corrections. Any one of the synthetic corrections and/or the simulated corrections may be generated by a computing unit.

The result of the one or more corrections to the first segmentation may be a second segmentation, which differs from the first segmentation, of the same medical image, e.g., at least locally at, and/or close to, the position of the one or more corrections by the user. E.g., the first segmentation and the segmentation may differ within a ROI of the medical image. The ROI may be specified by the user input.

The distance metric may also be denoted as, and/or may correspond to a, loss function.

The distance metric may comprise a Euclidean distance and/or a geodesic distance, or any combination thereof, e.g., a weighted sum.

The Euclidean distance may comprise a physical distance among pixels, e.g., among pixels with identical (in particular binary and/or K-ary) segmentation value and/or identical red green blue (RGB) and/or an RGB-alpha (RGBA) classification. Alternatively or in addition, the Euclidean distance may (e.g., completely) ignore the similarity between the pixels.

The geodesic distance may comprise a similarity measure between pixels in the neighbourhood, e.g., in terms of a (in particular binary and/or K-ary) segmentation value, and/or in terms of a color, e.g., according to a red green blue (RGB) and/or an RGB-alpha (RGBA) classification.

The distance metric may be applied, e.g., independently and/or separately, per pixel of the medical image.

The Euclidean distance and/or the geometric distance may be determined, e.g., independently and/or separately, for each pixel of the medical image.

In the training phase of the NN, the annotated segmentation (also denoted as the ground truth segmentation) may be, or may act as, a reference segmentation for determining the distance metric of the last (also denoted as latest) segmentation. Alternatively or in addition, the last-but-one (also denoted as preceding) segmentation may be, or may act, as the reference metric (and/or reference segmentation) for determining the distance metric of the last (also denoted as latest) segmentation.

In the inference phase (also denoted as application phase, and/or during deployment) of the NN, the distance metric may be determined relative to the one or more corrections by the user, and/or relative to a further (and/or modified) segmentation based on the one or more corrections by the user.

In the training phase, and/or in the inference phase, a reliability and/or a quality of the last segmentation may be quantified in terms of the distance metric. E.g., one or more small values of the distance metric may correspond to a reliable and/or high-quality segmentation. Alternatively or in addition, large values of the distance metric may correspond to an unreliable and/or low-quality segmentation.

Alternatively or in addition, training the NN, in particular comprising training phases without corrections by the user, may comprise applying a predetermined loss function, e.g., a cross-entropy loss function.

Still further alternatively or in addition, the distance metric may comprise a Dice score. By the Dice score, a similarity between the second segmentation (and/or the third, fourth, and/or any further, segmentation) and the annotated segmentation (also denoted as ground truth segmentation) may be determined (and/or quantified).

Any one of the loss functions, the distance metric, and/or the Dice score may comprise a weighted sum, e.g., a sum over pixels and/or a sum over weighted contributions. Alternatively or in addition, any one of the loss functions, the distance metric, and/or the Dice score may comprise a (e.g., independent) value per pixel of the medical image.

By performing the third segmentation based on the second segmentation, which comprises the one or more corrections by the user, and based on the determined distance metric, the NN (e.g., the CNN) may be trained to perform modifications to initial (e.g., first) segmentations based on user input (also denoted as corrections by the user).

Performing the third segmentation may comprise preserving the (first and/or) second segmentation at positions of the medical image, where the distance metric is below the predetermined threshold value. Alternatively or in addition, performing the third segmentation may comprise performing a new segmentation at positions of the medical image, where the distance metric is above the predetermined threshold value.

By training the NN on a plurality of medical images, in particular comprising a plurality of medical images obtained using a variety of medical imaging modalities, the NN may be able to perform segmentations, in particular in an inference phase (also denoted as application phase) on organs, and/or objects, that the NN has not been trained on in the training phase.

The inference phase (also: application phase) may comprise a phase of inference, e.g., segmentations, of medical images without annotated segmentations (and/or without available ground truth), and/or medical images that are not comprised in the training database.

Alternatively or in addition, by the training of the NN with corrections by a user, and/or with medical images from a variety of medical imaging modalities, the NN may be enabled for adaptation after the training phase, and/or for adaptation in the inference phase. The adaptivity may comprise (e.g., previously) untrained image contexts (e.g., in terms of the organs comprised in the medical image, in particular organs that were not comprised in the medical images of the training database), (e.g., previously) untrained pathologies (e.g., rare abnormalities, for which no suitable training dataset exists), and/or medical images from medical imaging modalities that were not represented in the training database.

The training method can enable a high segmentation accuracy and robustness, both for two-dimensional (2D) imaging and for three-dimensional (3D) imaging, in particular with a small number of user interactions in the inference phase, e.g., by only re-running a segmentation on, or close to, a position of the medical image, where the user performed one or more corrections (and/or only in a ROI indicated by the user input). Thereby, a time of the inference phase (also denoted as application phase) may be shortened, and/or a time of a user, e.g., a radiologist evaluating the medical image in view of a potential diagnosis and/or therapy plan, may be shortened. Alternatively or in addition, by the training method, a localized updating of the final segmentation, e.g., as compared to an initial (e.g., first) segmentation can be enabled, e.g. rather than an updating of the segmentation of the entire medical image. The extent of updating may be determined by the amount of user interaction (and/or user input, and/or corrections performed by the user).

Alternatively or in addition, by the computer-implemented method, both NN and user interaction-based uncertainties can be reduced during an image-specific fine-tuning, which the NN is trained on in the training phase and applies in the inference phase.

The method may further comprise, in particular in the training phase of the NN, a step of receiving, at the UI, at least one correction by the user on the output third segmentation.

Alternatively or in addition, the method may further comprise, in particular in the training phase of the NN, a step of determining, based on the output third segmentation, the fourth segmentation taking into account the at least one received correction to the third segmentation.

The steps of performing a further segmentation, e.g., by the NN, and receiving a further correction by the user may alternate. E.g., the training using the medical image of the first medical image dataset may repetitively perform the method steps until the segmentation output by the NN is sufficiently close to the annotated segmentation. The sufficient closeness may be measured, and/or determined, in terms of the distance metric, any one of the loss functions, and/or the Dice score.

The NN may comprise a convolutional neural network (CNN). Optionally, the NN and/or the CNN may comprise a U-net and/or one or more ResNet encoders.

Any one of the segmentations (e.g., the annotated segmentation - also denoted as ground truth segmentation, or briefly: ground truth -, the first segmentation, the second segmentation, the third segmentation, the fourth segmentation, and/or any further segmentation) may comprises a binary segmentation. The binary segmentation may comprise classifying each pixel of the medical image as object, in particular as organ, as background, and/or foreground.

By the binary segmentation, the medical image may be subdivided into regions of the image comprising at least part of an organ (e.g., only a part at the boundary of the image) and into regions not comprising (e.g., not comprising any part of) an organ.

A region not comprising (e.g., not comprising any part of) an organ may be denoted as background. Alternatively or in addition, a region comprising (e.g., any part of) an organ may be denoted as foreground. Further alternatively or in addition, the binary segmentation may comprise, e.g., the value 0 for any background pixel and the value 1 for any pixel comprising at least part of an organ (and/or for any foreground pixel), or vice versa.

Alternatively or in addition, the segmentation may distinguish between different types of organs. A K-ary segmentation may comprise, e.g., the value 0 for any background pixel and the values 1, 2, 3,..,K-1 for different types of organs, e.g., lung, liver, and/or kidney (and/or for different types of the foreground). Alternatively or in addition, the k values of the K-ary segmentation may comprise any permutation of values 0,1,..., k-1 for the different types of organs (and/or the foreground) and the background.

Any one of the binary values 0, 1 and/or the K-ary values 0, 1, 2,.., K-1 assigned to a pixel of the medical image may be denoted as label of the pixel.

Any classification as organ as presented herein may be generalized to a classification of objects. The objects may, e.g., comprise, organs, pathologies, and/or implants (e.g., a pacemaker, stent, and/or artificial joint). Alternatively or in addition, any organ according to the notation used herein may comprise a pathology and/or implant. Further alternatively or in addition, any object may comprise a segmentation protocol.

A segmentation protocol may comprise any quantity of interest to an (e.g., end-) user, e.g., any quantity visible and/or demandable on a medical image. The segmentation protocol may, e.g., comprise one or more organs, one or more pathologies, and/or one or more implants. Alternatively or in addition, the segmentation protocol may comprise one or more anatomical structures. Further alternatively or in addition, the segmentation protocol may comprise hardware and/or one or more foreign (e.g., relative to a patient's biological body) objects, e.g., one or more implants (for example comprising a pacemaker, stent, and/or artificial joint).

By the training phase incorporating one or more corrections by the user, the NN may be trained for flexibility in the inference phase, and/or for time efficiency. E.g., a segmentation in the inference phase may be obtained at interactive speed, e.g., ranging from a few seconds to several minutes.

Any one of the segmentations (e.g., the annotated segmentation and/or ground truth segmentation, the first segmentation, the second segmentation, the third segmentation, the fourth segmentation, and/or any further segmentation) may comprise a K-ary segmentation with K equal to, or greater than, three. The K-ary segmentation may comprise classifying each pixel of the medical image as background, as foreground, and/or as organ. Classifying a pixel as organ (and/or as foreground) may further comprise classifying a type of organ (and/or a type of foreground).

The at least one correction by the user on any one of the segmentations (e.g., on the first segmentation, on the third segmentation, and/or on any further segmentation) may comprise a manual correction performed by the user.

The manual correction may comprise a, e.g., predetermined, part of the annotated segmentation (also denoted as ground truth segmentation). Alternatively or in addition, the first medical image dataset may comprise one or more manual corrections stored separately for the training phase.

Alternatively or in addition, the at least one correction by the user on any one of the segmentations (e.g., on the first segmentation, on the third segmentation, and/or on any further segmentation) may comprises a simulated correction.

The simulated correction may also be denoted as synthetic corrections. Alternatively or in addition, the simulated correction may comprise a correction generated by a computer algorithm, e.g., based on a differences between the annotated segmentation (also denoted as ground truth) and the first segmentation, e.g., close to the annotated segmentation, and/or interpolating between the first, third, and/or any further segmentation performed by the NN and the annotated segmentation.

The at least one correction by the user may comprise at least a piece of a contour, in particular of an object. Alternatively or in addition, the at least one correction by the user may comprise at least a piece of a border, in particular between objects. Alternatively or in addition, the at least one correction by the user may comprise a selection as interior of an object. Further alternatively or in addition, the at least one correction by the user may comprise a selection as exterior of an object. Still further alternatively or in addition, the at least one correction by the user may comprise a marking of a position.

The contour, and/or the border, may also be denoted as boundary.

The one or more corrections by the user may comprise drawing the contour, boundary, and/or border. Alternatively or in addition, the one or more corrections by the user may comprise marking a position (also denoted as location), and/or a ROI, on the medical image. The marking may comprise assigning a property to the position and/or to the ROI. The property may e.g., comprise if the position, and/or the ROI, belongs to the background, belongs to the foreground, forms part of an organ, and/or is assigned a color, e.g., according to the RGB and/or the RGBA scheme. Further alternatively or in addition, the one or more corrections by the user may comprise painting a position and/or painting a ROI.

Alternatively or in addition, the at least one correction by the user on any one of the segmentations (e.g., on the first segmentation, on the third segmentation, and/or on any further segmentation) may be performed on a localized ROI. Optionally, the second segmentation, the fourth segmentation, and/or any further segmentation based on the at least one correction by the user only differs from the first segmentation, the third segmentation, and/the preceding further segmentation output by the NN, respectively, only within, or near, the ROI.

By only re-running the segmentation (e.g., algorithm) along the ROI, the segmentation (e.g., algorithm) may be sped up, in particular in the inference phase. Alternatively or in addition, computing resources may be saved by only re-running the segmentation (e.g., algorithm) along parts of the medical image (e.g., only along one or more ROIs marked by the user input).

The medical image may comprise a two-dimensional (2D) image. Alternatively or in addition, the medical image may comprise a three-dimensional (3D) image, in particular a stack of slices.

The medical image may comprise a two-dimensional image, e.g., an X-ray. Alternatively or in addition, the medical image may comprise a stack of slices, e.g., obtained by MRI and/or CT. Further alternatively or in addition, the medical image may comprise a temporal sequence of instantaneous medical images. Any one of the instantaneous medical images may comprise at least one two-dimensional image (e.g., a stack of slices) and/or a three-dimensional image.

The medical image may be acquired from a medical imaging device using a medical image modality comprising MRI, CT, US, PET, single-photon emission computed tomography (SPECT), angiography, and/or radiography.

Radiography may also be denoted as X-ray imaging.

The distance metric may comprise a Euclidean metric and/or a geodesic metric. Optionally, the distance metric may comprise a, in particular weighted, sum of the Euclidean metric and the geodesic metric.

The distance metric may also be denoted as distance map. Alternatively or in addition, the distance metric may be specific to, e.g., have an independent value for, each pixel of the medical image.

The weighted sum may interpolate between a purely Euclidean metric and a purely geodesic metric.

Training the NN may comprise applying any one of the preceding method steps to a plurality, or all, first medical image datasets comprised in the training database.

The method may further comprise an inference phase. The inference phase may comprise receiving, at the input layer of the NN, a further medical image from a medical imaging device. The inference phase may further comprise performing, by the one or more hidden layers of the NN with interconnections, a segmentation of anatomical structures comprised in the received further medical image. The interconnections may have been determined in the training phase of the NN. The inference phase may still further comprise outputting the segmentation of the anatomical structures of the further medical image.

The further medical image may also be denoted as up-to-date medical image and/or as diagnostic medical image. Alternatively or in addition, the further medical image may not have an associated ground truth segmentation.

The inference phase may further comprise receiving one or more corrections by a user and modifying the output segmentation (e.g., according to the one or more corrections by the user). E.g., the modification of the output segmentation may comprise performing a new segmentation, in particularly locally close to, and/or within a bounding box around, the corrections by the user. Alternatively or in addition, modifying the output segmentation may comprise iterations, e.g., based on determining a distance metric between the latest segmentation and the preceding segmentation. For example, the iterations may be continued based on the distance metric exceeding a predetermined threshold, and/or based on user input, e.g., initiating a further segmentation step.

By the method, modifying the output segmentation may be performed locally, e.g., only on a small fraction of the further medical image. Thereby, computing resources and/or memory may be saved.

As to a system aspect, a neural network system for training a NN for interactive medical image segmentation is provided. The system comprises the NN. The NN comprises an input layer configured to receive a medical image of a first medical image dataset from a training database of medical image datasets as input to the NN, wherein the first medical image dataset comprises the medical image and optionally an annotated segmentation (which may also be denoted as ground truth segmentation). The NN further comprises one or more hidden layers with interconnections configured to perform a first segmentation of anatomic structures comprised in the medical image of the received first medical image dataset. The NN still further comprises an output layer configured to output the performed first segmentation for corrections by a user.

The system further comprises a UI configured to receive at least one correction by the user on the output first segmentation.

The system further comprises a determining unit configured to determine, based on the output first segmentation, a second segmentation taking into account the at least one received correction to the first segmentation.

The system further comprises an annotation reception interface configured to receive the annotated segmentation of the first medical image dataset. Alternatively or in addition, the input layer of the NN may comprise the annotation reception interface for receiving the annotated segmentation of the first medical image dataset.

The system further comprises a computing unit configured to determine a distance metric between the determined second segmentation and the annotated segmentation comprised in the received medical image dataset in association with the medical image. Alternatively or in addition, the computing unit is configured to determine a distance metric between the determined second segmentation and the output first segmentation.

The system still further comprises a combining unit configured to combine the determined distance metric, the determined second segmentation and the medical image (302) into a second medical image dataset.

The input layer of the NN is further configured to receive the combined second medical image dataset, and/or at least the medical image comprised in the second medical image dataset, as input to the NN.

The one or more hidden layers of the NN with interconnections are further configured to perform a third segmentation of the anatomic structures comprised in the medical image of the received second medical image dataset. Performing the third segmentation comprises changing one or more weights of the interconnections of the NN. The changing of the one or more weights of the interconnections of the NN is based on the determined distance metric comprised in the second medical image dataset.

The output layer of the NN is further configured to output the performed third segmentation.

The computing unit is further configured to determine a further distance metric between the output third segmentation, and/or a fourth segmentation based on the third segmentation, and the annotated segmentation comprised in the received second medical image dataset. Alternatively or in addition, the computing unit is further configured to determine a further distance metric between the output third segmentation and the determined second segmentation, and/or a further distance metric between the (e.g., determined, in particular responsive to one or more further corrections by the user) fourth segmentation and the output third segmentation.

The NN is further configured to selectively repeat at least the steps of receiving a combined further medical image data set, performing a further segmentation of the anatomic structures comprised in the medical image of the received further medical image data set, outputting the performed further segmentation, and/or determining a (e.g., still) further distance metric between the output further segmentation and the annotated segmentation. Alternatively or in addition, the (e.g., still) further distance metric is determined between the output further segmentation and the directly preceding segmentation.

The selectivity of the step of selectively repeating the steps may comprise determining to repeat at least some of the previous steps if the further distance metric, or any still further distance metric, exceeds a predetermined threshold. Alternatively or in addition, the selectivity of the step of selectively repeating at least some of the previous steps may comprise determining to not repeat the steps if the further distance metric, or any still further distance metric, falls below the predetermined threshold.

The neural network system may be configured to perform any one of the steps, or comprise any one of the features, of the method aspect.

As to a further aspect, a computer program product is provided comprising program elements which induce a neural network system to carry out the steps of the method aspect for training a NN for interactive medical image segmentation, when the program elements are loaded into a memory of the neural network system.

As to a still further aspect, a computer-readable medium is provided on which program elements are stored that can be read and executed by a neural network system, in order to perform steps of the method aspect for training a NN for interactive medical image segmentation, when the program elements are executed by the neural network system.

The properties, features and advantages of this invention described above, as well as the manner they are achieved, become clearer and more understandable in the light of the following description and embodiments, which will be described in more detail in the context of the drawings. This following description does not limit the invention on the contained embodiments. Same components or parts can be labelled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

Fig. 1         is a flow chart of a computer-implemented method for training a neural network (NN) for interactive medical image segmentation according to a preferred embodiment of the present invention;

Fig. 2      is an overview of the structure and architecture of a NN system according to a preferred embodiment of the present invention;

Fig. 3      shows an exemplary embodiment of the method of Fig. 1;

Fig. 4      schematically illustrates a visual comparison between a first segmentation and a second segmentation based on a correction by a user; and

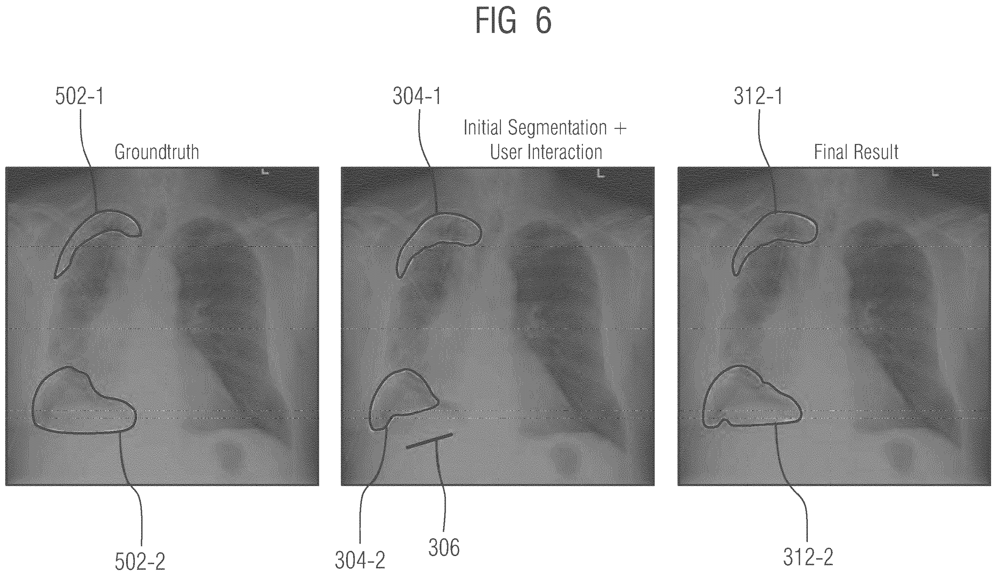

Figs. 5 and 6      schematically illustrate visual comparisons between a first segmentation, a second segmentation based on a correction by a user and a ground truth segmentation, wherein Fig. 5 shows a segmentation of the lung and Fig. 6 shows a segmentation of potential pathologies.

Any reference signs in the claims should not be construed as limiting the scope.

DETAILLED DESCRIPTION

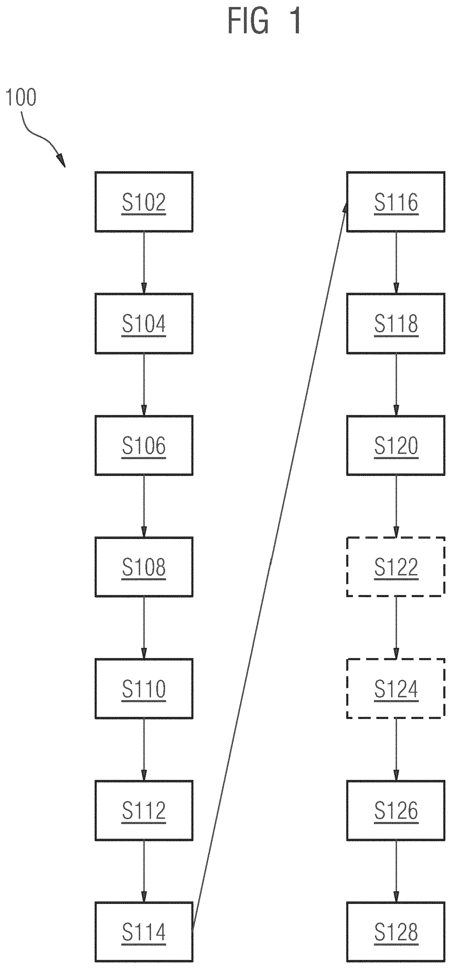

Fig. 1 shows an exemplary flow chart of a computer-implemented method for training a neural network (NN) for interactive medical image segmentation. The method is generally denoted by reference sign 100.

The method 100 comprises a training phase of the NN.

In a step S102 of the training phase of the NN, a first medical image dataset is received at an input layer of the NN from a training database of medical image datasets as input to the NN. The first medical image dataset comprises a medical image and optionally an annotated segmentation (which may also be denoted as ground truth segmentation, or briefly as ground truth).

In a step S104 of the training phase of the NN, a first segmentation of anatomic structures comprised in the medical image of the received S102 first medical image dataset is performed by one or more hidden layers of the NN with interconnections.

In a step S106 of the training phase of the NN, the performed S104 first segmentation is output, at an output layer of the NN, for corrections by a user.

In a step S108 of the training phase of the NN, at least one correction by the user on the output S106 first segmentation is received at a user interface (UI).

In a step S110 of the training phase of the NN, based on the output S106 first segmentation, a second segmentation is determined taking into account the at least one received S108 correction to the first segmentation.

In a step S112 of the training phase of the NN, a distance metric between the determined S110 second segmentation and the annotated segmentation (also denoted as ground truth segmentation) comprised in the received S102 medical image dataset in association with the medical image is determined. Alternatively or in addition, the step S112 of determining the distance metric comprises determining a distance metric between the determined S110 second segmentation and the output first segmentation S106.

In a step S114 of the training phase of the NN, the determined S112 distance metric, the determined S110 second segmentation and the medical image are combined into a second medical image dataset.

In a step S116 of the training phase of the NN, the combined S114 second medical image dataset is received, at the input layer of the NN, as input to the NN.

In a step S118 of the training phase of the NN, a third segmentation of the anatomic structures comprised in the medical image of the received S116 second medical image dataset is performed by the one or more hidden layers of the NN with interconnections. Performing S118 the third segmentation comprises changing one or more weights of the interconnections of the NN. The changing of the one or more weights of the interconnections of the NN is based on the determined S112 distance metric comprised in the second medical image dataset.

In a step S120 of the training phase of the NN, the performed S118 third segmentation is output at the output layer of the NN.

In a step S126 of the training phase of the NN, a further distance metric between the output S120 third segmentation, and/or a fourth segmentation based on the third segmentation (e.g., after one or more further corrections by the user), and the annotated segmentation comprised in the received S116 second medical image dataset is determined. Alternatively or in addition, the step S126 of determining the further distance metric comprises determining a further distance metric between the output S120 third segmentation and the determined S110 second segmentation, and/or a further distance metric between the (e.g., determined, in particular responsive to one or more further corrections by the user) fourth segmentation and the output S120 third segmentation.

In a step S128 of the training phase of the NN, any of the preceding steps may be selectively repeated. The preceding steps that may be repeated may in particular comprise receiving S116 a combined further medical image data set, performing S118 a further segmentation of the anatomic structures comprised in the medical image of the received S116 further medical image data set, outputting S120 the performed further segmentation, and/or determining S126 a (e.g., still) further distance metric between the output S120 further segmentation and the annotated segmentation. Alternatively or in addition, the (e.g., still) further distance metric is determined between the output S120 further segmentation and the directly preceding segmentation.

The selectivity of the step S128 of selectively repeating comprises determining to repeat the steps (e.g., S116, S118, S120, and/or S126) if the further distance metric, or any still further distance metric, exceeds a predetermined threshold. Alternatively or in addition, the selectivity of the step S128 of selectively repeating comprises determining to not repeat the steps (e.g., S116, S118, S120, and/or S126) if the further distance metric, or any still further distance metric, falls below the predetermined threshold.

Optionally, in a step S122 of the training phase of the NN, at least one correction by the user on the output S120 third segmentation is received at the UI.

Further optionally, in a step S124 of the training phase of the NN, based on the output S120 third segmentation, the fourth segmentation is determined taking into account the at least one received S122 correction to the third segmentation.

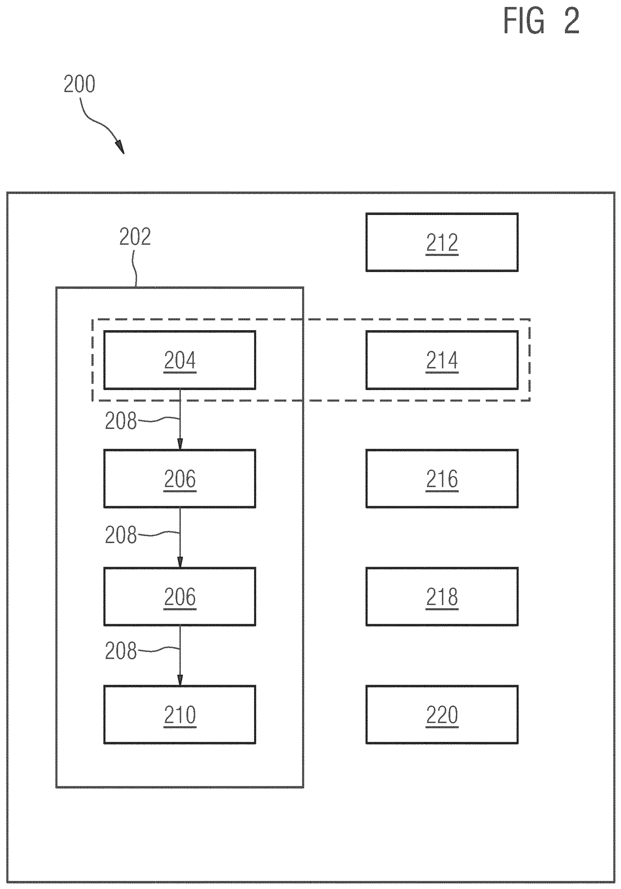

Fig. 2 shows a neural network system for training a neural network (NN) for interactive medical image segmentation. The NN system is generally referred to by reference sign 200, and the NN is generally referred to by reference sign 202.

The NN 202 comprises an input layer 204 configured to receive a first medical image dataset from a training database of medical image datasets as input to the NN 202. The first medical image dataset comprises a medical image and optionally an annotated segmentation (which may also be denoted as ground truth segmentation).

The NN 202 further comprises one or more hidden layers 206 with interconnections 208 configured to perform a first segmentation of anatomic structures comprised in the medical image of the received first medical image dataset. In Fig. 2, merely as illustrative example, two hidden layers 206 with one or more interconnections 208 among the hidden layers as well as interconnections 208 with the input layer 204 and the output layer 210 are shown.

The NN 202 still further comprises an output layer 210 configured to output the performed first segmentation for corrections by a user.

The NN system 200 further comprises a user interface (UI) 212 configured to receive at least one correction by the user on the output first segmentation.

The NN system 200 further comprises a determining unit 216 configured to determine, based on the output first segmentation, a second segmentation taking into account the at least one received correction to the first segmentation.

The NN system 200 further comprises an annotation reception interface 214 configured to receive the annotated segmentation of the first medical image dataset.

The NN system 200 further comprises a computing unit 218 configured to determine a distance metric between the determined second segmentation and the annotated segmentation comprised in the received medical image dataset in association with the medical image. Alternatively or in addition, the distance metric is determined, by the computing unit 218, between the determined second segmentation and the output first segmentation.

The NN system 200 still further comprises a combining unit 220 configured to combine the determined distance metric, the determined second segmentation and the medical image into a second medical image dataset.

The input layer 204 of the NN 202 is further configured to receive the combined second medical image dataset as input to the NN 202. The one or more hidden layers 206 of the NN 202 with interconnections 208 are further configured to perform a third segmentation of the anatomic structures comprised in the medical image of the received second medical image dataset. Performing the third segmentation comprises changing one or more weights (not shown in Fig. 2) of the interconnections 208 of the NN 202. The changing of the one or more weights of the interconnections 208 of the NN 202 is based on the determined distance metric comprised in the second medical image dataset. The output layer 210 of the NN 202 is further configured to output the performed third segmentation.

The computing unit 218 is further configured to determine a further distance metric between the output third segmentation, and/or a fourth segmentation based on the third segmentation (e.g., based on one or more further corrections by the user), and the annotated segmentation comprised in the received second medical image dataset. Alternatively or in addition, the further distance metric is determined, by the computing unit 218, between the output third segmentation and the determined second segmentation, and/or between the (e.g., determined, in particular responsive to one or more further corrections by the user) fourth segmentation and the output third segmentation.

The NN 202 is further configured to selectively repeat at least the steps of receiving a combined further medical image data set, performing a further segmentation of the anatomic structures comprised in the medical image of the received further medical image data set, outputting the performed further segmentation, and determining a (e.g., still) further distance metric between the output further segmentation and the annotated segmentation. Alternatively or in addition, the (e.g., still) further distance metric is determined between the output further segmentation and the directly preceding segmentation.

The selectivity of the step of selectively repeating the (e.g., previous) steps may comprise determining to repeat the (e.g., previous) steps if the further distance metric, or any still further distance metric, exceeds a predetermined threshold. Alternatively or in addition, the selectivity of the step of selectively repeating the (e.g., previous) steps may comprise determining to not repeat the (e.g., previous) steps if the further distance metric, or any still further distance metric, falls below the predetermined threshold.

In Fig. 2, the input layer 204 of the NN 202 and the annotation reception interface 214 are shown as separate entities. As indicated by the dashed line, the input layer 204 of the NN 202 and the annotation reception interface 214 may be merged in a joint entity, which may also be denoted as input layer.

In Fig. 2, the determining unit 216, the computing unit 218 and the combining unit 220 are further shown as separate entities. Any one of these entities may be combined with other entities shown in Fig. 2. For example, the computing unit 218 and the combining unit 220 may be realised by a processing unit, optionally further comprising memory.

Alternatively or in addition, the determining unit 216 may be comprised in the NN 202. E.g., the second segmentation may be determined by making use of the NN 202 for modifying the first segmentation according to the one or more corrections by the user.

Alternatively or in addition, the determining unit 216 may be realised by the processing unit, optionally further comprising memory. E.g., the corrections by the user may comprise a change in a contour with respect to the first segmentation. The second segmentation may correspond to deleting the previous contour and inserting the changed contour on the first segmentation.

The invention improves on conventional medical image segmentation techniques in an at least four-fold way.

Firstly, a novel deep learning (DL)-based framework for interactive two-dimensional (2D) and three-dimensional (3D) medical image segmentation is provided by incorporating NNs, in particular CNNs, into a binary segmentation pipeline. Binary herein may refer to only distinguishing a background from a foreground and/or an organ displayed on the medical image.

Secondly, an image-specific fine-tuning is used to adapt a NN, in particular a CNN, model to each test image independently, preferably for enabling an improved segmentation. The fine-tuning may be unsupervised (e.g., without additional user interactions), and/or supervised, e.g., where user-provided corrections (also denoted as scribbles) will guide the learning process. The user-provided corrections (and/or scribbles) may comprise a user drawing a contour, e.g., determining a boundary of an object, a user drawing a contour marking an interior and/or exterior of an object of interest, and/or any other form, e.g., comprising a pointer to a region of interest (ROI). The ROI may indicate, e.g., image pixels, where the fine-tuning, e.g., the third segmentation or any further segmentation, is to be performed.

Thirdly, a weighted loss function may be employed for considering network and/or interaction-based uncertainties during image-specific fine-tuning, e.g., when performing the third segmentation or any further segmentation.

Fourthly, by the inventive technique, NNs, in particular CNNs, are enabled (e.g., in an inference phase and/or after a training phase) to segment previously unseen objects. Alternatively or in addition, the inventive technique does not require annotations of all the organs for the training phase. The NN, in particular comprising a CNN, may be applied to new (e.g., not comprised in the training database) organs, pathologies, and/or new segmentation protocols directly, e.g., in the inference phase. Further alternatively or in addition, by the training of the NN comprising user corrections, different organs and/or different imaging modalities in the training phase, in the inference phase the NN may be enabled to perform segmentations of new (and/or never-before-seen) organs and/or anatomical regions (e.g., one or more organs and/or anatomical regions without any ground truth segmentation and/or without any medical image dataset in the training database).

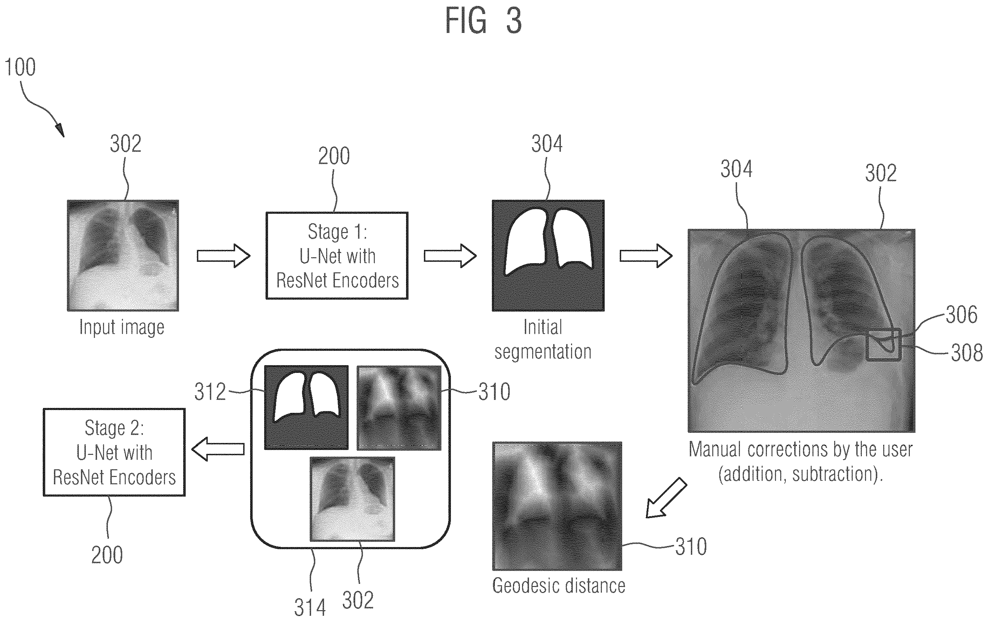

An exemplary embodiment of the inventive method 100 for interactive medical image segmentation is depicted in Fig. 3.

A medical image 302 is provided to the NN system 200, in particular to the input layer 204 of the NN 202 of Fig. 2. The exemplary NN 202 in Fig. 3 comprises a U-net with ResNet encoders as CNN. At reference sign 304, a first segmentation (also denoted as initial segmentation) is output, e.g., by the output layer 210 of the NN 202 of Fig. 2.

In the example of Fig. 3, the medical image 302 is displayed with the first segmentation 304 overlayed, e.g., on the UI 212 of Fig. 2. A correction 306 by the user defines a bounding box (and/or ROI) 308, along which the first segmentation 304 is considered as incorrect by the user.

At reference sign 310, a distance metric, e.g., per image pixel, is determined. The distance metric may in particular comprise a geodesic distance.

A second medical image dataset 314 comprises the (in particular original) medical image 302, the distance metric 310 and the second segmentation 312. Alternatively or in addition, the second medical image dataset 314 may comprise the first segmentation 304, the distance metric 310 and the medical image 302. By the distance metric 310, the first segmentation 304 may be recovered from the second segmentation 312, or vice versa.

The second medical image dataset 314 is provided to the input layer 204 of the NN 202, in particular the CNN, for performing the third segmentation. In the exemplary embodiment of Fig. 3, the NN 202 comprise a U-net with ResNet encoders.

During the training phase (which may also be denoted as training stage, test phase, and/or test stage), at least one correction (e.g., defining and/or comprising a bounding box) is provided by the user, and the segmentation and the NN, in particular the CNN, are alternatively refined through unsupervised (e.g., without additional user interactions), and/or supervised (e.g., with user-provided corrections and/or scribbles) image-specific fine-tuning.

Unlike in conventional techniques, the refinement of the contour may occur in the localized ROI, e.g., defined by the bounding box 308 in Fig. 3. The contour outside the ROI, e.g., outside the bounding box 308 in Fig. 3, may remain untouched. Alternatively or in addition, the first segmentation 304, the second segmentation 312, and/or any further segmentation may coincide outside the localized ROI, e.g., outside the bounding box 308 in Fig. 3.

The size and dimensions of the ROI may be determined based on the corrections by the user (also denoted as user interaction). E.g., larger corrections (and/or scribbles) may result in a large ROI.

The inventive technique is general, flexible and can handle both two-dimensional (2D) and three-dimensional (3D) segmentations with few assumptions of network structures.

The example of Fig. 3 shows a two-dimensional (2D) medical image for illustrative purposes of the training phase (which may also be denoted as testing phase). The change of the segmentations within the exemplary bounding box 308 may also be denoted as image-specific fine tuning. The distance metric 310 may also be denoted as, in particular weighted, loss function. The loss function may be weighted, e.g., in the sense that the distance metric 310 interpolates between a geodesic distance metric and a Euclidean distance metric.

The illustrative example of Fig. 3 shows a binary segmentation 304; 312 of a two-dimensional (2D) medical image comprising the lung as organ and/or foreground, and otherwise background, in the training phase (also denoted as training stage) of the NN 202.

More generally, the first segmentation (also denoted as initial segmentation) may comprise a two-dimensional (2D) segmentation and/or a three-dimensional (3D) segmentation of a two-dimensional (2D) and/or a three-dimensional (3D) medical image, respectively. Alternatively or in addition, the segmentation may be binary and/or K-ary with K bigger or equal to three.

A K-ary segmentation training dataset may comprise a set of pairs, e.g., $T = \{(X1;\ Y1);\ (X2;\ Y2);\ ...\}$ where $Xp$ is one training medical image and $Yp$ is the corresponding label map. The label set of $T$ may be $\{0, 1, 2, ... , K - 1\}$ with 0 being the background label and $\{1, 2, ... , K - 1\}$ corresponding to different foreground and/or organ labels. $N_k$ may denote the number of instances of the k-th object (e.g., organ and/or foreground) type. The total number of instances may be $\hat{N} = \Sigma_k N_k$. Each image $Xp$ may have instances of multiple object classes (e.g., comprise at least two organs and/or different foreground components). The label of the $q$-$th$ instance in $Xp$ may be denoted as $I_{pq}$. $Y_p$ may be converted into a binary (and/or K-ary) image $Y_{pq}$ based on whether the value of each pixel in $Y_p$ equals to $I_{pq}$. Subsequently, a NN 202, in particular a CNN (e.g., comprising a U-Net), may be trained to extract the binary and/or K-ary segmentation, and/or to extract at least one object and/or one organ (also denoted as target). Extracting one object and/or one organ may be based on a binary segmentation problem irrespective of the object type and/or the organ type.

A cross-entropy loss function may be used for the training of the NN. Alternatively or in addition, at least at locations of one or more corrections performed by a user, a distance metric may be used for the training of the NN.

The corrections by a user may comprise manual corrections and/or synthetically generated corrections (which may also be denoted as simulated corrections). The synthetically generated corrections may, e.g., be based on (in particular a localized region of) the annotated segmentation (also denoted as ground truth segmentation). Alternatively or in addition, the corrections may be denoted as image-specific label refinement, e.g., based on geodesic maps.

With the initial (e.g., first) segmentation $\hat{Y}_0$ obtained by the (e.g., at least partially trained) NN, in particular the CNN, the user may provide a (e.g., supervised) set of corrections (and/or scribbles) to guide the update of the segmentation $\hat{Y}_0$. $S^f$ and $S^b$ may denote the corrections (and/or scribbles) for foreground and background, respectively. The entire set of corrections (and/or scribbles) may be $S = S^f \cup S^b$. $s_i$ may denote the user-provided label of a pixel in the corrections (and/or scribbles). According to a choice of convention, $s_i = 1$ if $i \in S^f$ and $s_i = 0$ if $i \in S^b$ may be assigned. According to a different choice of convention, the values 1 and 0 may be reversed, i.e., 0 for the foreground and 1 for the background.

Interactions with the same label may be converted into a distance metric (also denoted as distance map) that may be the weighted sum of both the Euclidean and geodesic distance:

$$D = \alpha D_{Euclidean} + (1 - \alpha) D_{Geodesic}$$

with the parameter $\alpha$ interpolating between a purely Euclidean distance and a purely geodesic distance.

In Fig. 3 at reference sign 310, an example of geodesic distance metric (also denoted as distance transforms or distance map) of user interactions is shown.

The (e.g., geodesic) distance metric (also denoted as distance map) 310 of user interactions and the first segmentation (also denoted as initial automatic segmentation) 304 may have the same size (e.g., an identical number of pixels and/or digital dimensions) as the medical image 302.

The (e.g., geodesic) distance metric 310 and the first segmentation 304 may be concatenated with the raw channels of the medical image 302 so that a concatenated image with $C_l + 3$ channels is obtained, which is used as the input of the refinement network. The concatenated image may correspond to the second medical image dataset 314 of Fig. 3.

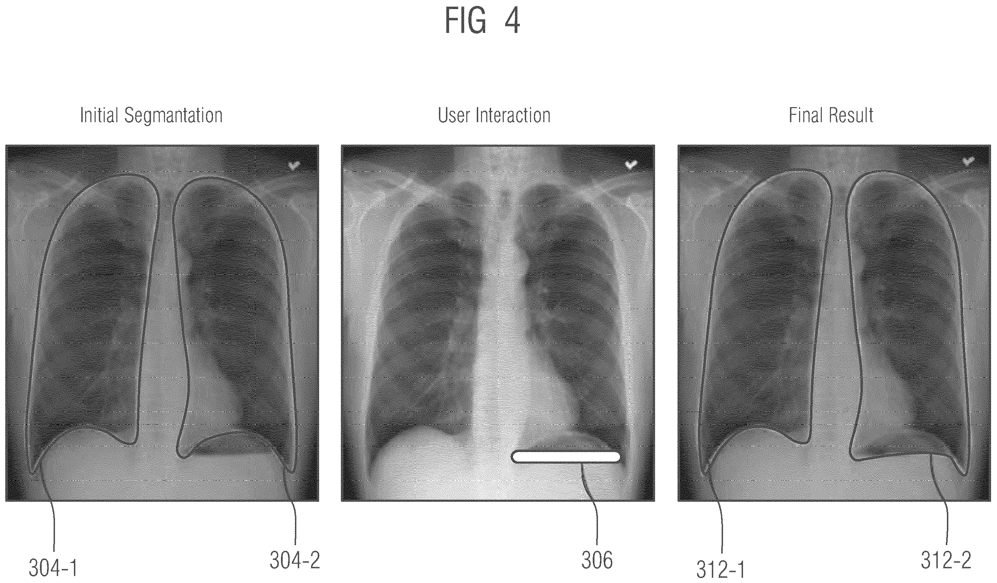

Fig. 4 shows an example of a visual comparison of the first (and/or initial) segmentation 304-1; 304-2 of the lung and second (and/or updated) segmentation 312-1; 312-2 using the inventive DL technique for editing lung-field segmentation in a two-dimensional (2D) chest X-ray (CXR). User-edits and/or corrections by the user (e.g., addition in the example at hand) are shown at reference sign 306 in the middle image. In the example of Fig. 4, only the part of the lung on the right-hand side in each image is modified from 304-2 to 312-2, whereas the first segmentation 304-1 and the second segmentation 312-1 of the part of the lung on the left hand side of each image are identical.

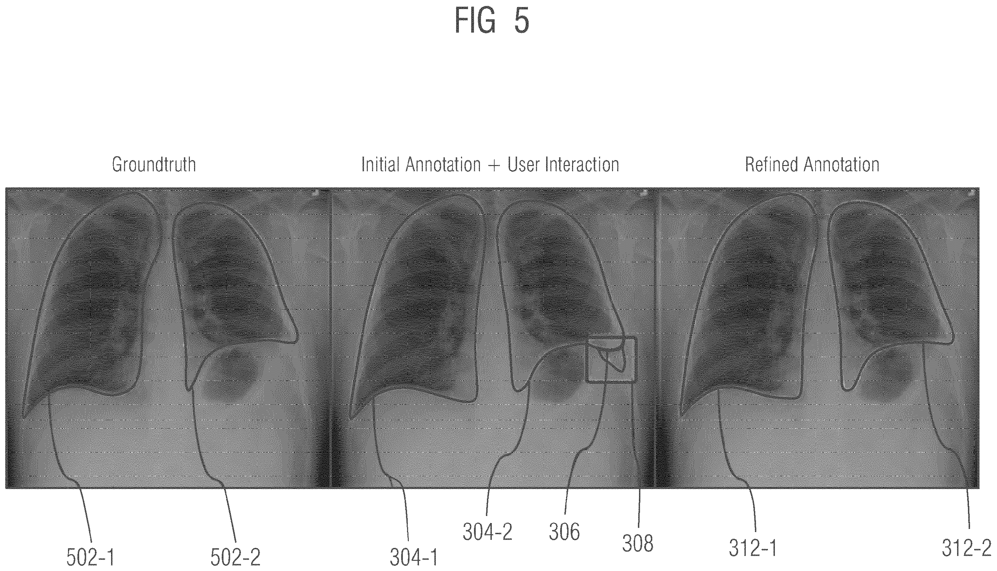

Fig. 5 shows an exemplary visual comparison of the first (and/or initial) segmentation 304-1; 304-2 and the second (and/or updated) segmentation 312-1; 312-2 using the inventive DL technique for editing lung-field segmentation in a two-dimensional (2D) chest X-ray (CXR). User-edits and/or corrections (e.g., deletion in the example at hand) are shown at reference sign 306 in the middle image along with a bounding box and/or ROI 308. For the sake of comparing the improvement in the segmentation 312-2 due to the correction 306, on the left image, the annotated segmentation (also denoted as ground truth segmentation) 502-1; 502-2 is shown. As the correction 306 by the user is only performed on the part of the lung on the right-hand side of each image, the first segmentation 304-1 and the second segmentation 312-1 of the left-hand side of the lung agree. Alternatively or in addition, no significant deviation of the segmentations 304-1 and 312-1 of the part of the lung on the left hand side of each image from the annotated segmentation 502-1 is noticeable.

Fig. 5 shows an exemplary visual comparison of the first (and/or initial) segmentation 304-1; 304-2 and the second (and/or updated) segmentation 312-1; 312-2 for a pathology (e.g., pneumothorax) using the inventive DL framework in a two-dimensional (2D) chest X-ray (CXR). One or more user-edits and/or corrections (e.g., an addition in the case at hand, in particular to the part 304-2 of the first segmentation) is shown at reference sign 306 in the middle image. By comparing the annotated segmentation (also denoted as ground truth segmentation) 502-1; 502-2 in the left image of Fig. 5 with the second segmentation 312-1; 312-2, the improvement due to the correction 306 is visible compared to the first segmentation 304-1; 304-2.

The inventive technique provides an efficient deep learning-based framework for interactive two-dimensional (2D) and/or three-dimensional (3D) medical image segmentation which can be faster and optimal compared to conventional manual-editing tools.

Efficient manual editing can enable a stronger clinical application and acceptance by the user (e.g., a medical practitioner) compared to fully automated segmentation techniques, which do not allow editing and whereby the user has to accept or reject a fully automated segmentation completely. This is particularly relevant if the fully automated segmentation only deviates at localized regions from a segmentation performed by the user. The localized regions can be of particular importance for detecting and/or analyzing anomalies and/or potential pathologies.

The inventive technique provides a time-efficient and labor-efficient method for producing high-quality annotations for training NNs. In particular simulated corrections, e.g., generated based on the annotated segmentation of the corresponding medical image, can provide improvements in the training of a NN for interactive segmentation.

Same corrections (and/or same scribbles) are expected to produce the same (e.g., final) segmentation (and/or annotation). A Dice score (also known as Dice Similarity Coefficient) can be used to check the overlap (and/or the similarity) of two segmentations (and/or annotations).

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

List of References

[1] Rajchl, M., Lee, M. C., Oktay, O., Kamnitsas, K., Passerat-Palmbach, J., Bai, W., ... & Rueckert, D. (2016). Deepcut: Object segmentation from bounding box annotations using convolutional neural networks. IEEE transactions on medical imaging, 36(2), 674-683.

[2] Criminisi, A., Sharp, T., & Blake, A. (2008, October). Geos: Geodesic image segmentation. In European Conference on Computer Vision (pp. 99-112). Springer, Berlin, Heidelberg.

[3] Rother, C., Kolmogorov, V., & Blake, A. (2004). "GrabCut" interactive foreground extraction using iterated graph cuts. ACM transactions on graphics (TOG), 23(3), 309-314.

[4] Wang, B., Liu, K. W., Prastawa, K. M., Irima, A., Vespa, P. M., Van Horn, J. D., ... & Gerig, G. (2014, April). 4D active cut: An interactive tool for pathological anatomy modeling. In 2014 IEEE 11th International Symposium on Biomedical Imaging (ISBI) (pp. 529-532). IEEE.

[5] Girshick, R., Donahue, J., Darrell, T., & Malik, J. (2014). Rich feature hierarchies for accurate object detection and semantic segmentation. In Proceedings of the IEEE conference on computer vision and pattern recognition (pp. 580-587).

## Claims

1. Computer-implemented method (100) for training a neural network, NN (202), for interactive medical image segmentation, a training phase of the NN (202) comprising the method steps of:

   - Receiving (S102), at an input layer (204) of the NN (202), a first medical image dataset from a training database of medical image datasets as input to the NN (202), wherein the first medical image dataset comprises a medical image (302) and optionally an annotated segmentation (502-1; 502-2);
   - Performing (S104), by one or more hidden layers (206) of the NN (202) with interconnections (208), a first segmentation (304; 304-1; 304-2) of anatomic structures comprised in the medical image (302) of the received (S102) first medical image dataset;
   - Outputting (S106), at an output layer (210) of the NN (202), the performed (S104) first segmentation (304; 304-1; 304-2) for corrections (306) by a user;
   - Receiving (S108), at a user interface, UI (212), at least one correction (306) by the user on the output (S106) first segmentation (304; 304-1; 304-2);
   - Determining (S110), based on the output (S106) first segmentation (304; 304-1; 304-2), a second segmentation (312; 312-1; 312-2) taking into account the at least one received (S108) correction (306) to the first segmentation (304; 304-1; 304-2);
   - Determining (S112) a distance metric (310) between the determined (S110) second segmentation (312; 312-1; 312-2) and the annotated segmentation (502-1; 502-2) comprised in the received (S102) medical image dataset in association with the medical image (302) and/or between the determined (S110) second segmentation (312; 312-1; 312-2) and the output (S106) first segmentation (304; 304-1; 304-2);
   - Combining (S114) the determined (S112) distance metric (310), the determined (S110) second segmentation (312; 312-1; 312-2) and the medical image (302) into a second medical image dataset (314);
   - Receiving (S116), at the input layer (204) of the NN (202), the combined (S114) second medical image dataset (314) as input to the NN (202);
   - Performing (S118), by the one or more hidden layers (206) of the NN (202) with interconnections (208), a third segmentation of the anatomic structures comprised in the medical image of the received (S116) second medical image dataset (314), wherein performing (S118) the third segmentation comprises changing one or more weights of the interconnections (208) of the NN (202), wherein the changing of the one or more weights of the interconnections (208) of the NN (202) is based on the determined (S112) distance metric (310) comprised in the second medical image dataset (314);
   - Outputting (S120), at the output layer (210) of the NN (202), the performed (S118) third segmentation;
   - Determining (S126) a further distance metric between the output (S120) third segmentation, and/or a fourth segmentation based on the third segmentation, and the annotated segmentation (502-1; 502-2) comprised in the received (S116) second medical image dataset (314) and/or between the output (S120) third segmentation, and/or the fourth segmentation based on the third segmentation, and the determined (S110) second segmentation (312; 312-1; 312-2), and/or the output (S120) third segmentation; and
   - Selectively repeating (S128) at least the steps of receiving (S116) a combined further medical image data set, performing (S118) a further segmentation of the anatomic structures comprised in the medical image (302) of the received (S116) further medical image data set, outputting (S120) the performed further segmentation, and determining (S126) a still further distance metric between the output (S120) further segmentation and the annotated segmentation (502-1; 502-2) and/or between the output (S120) further segmentation and the directly preceding segmentation;

   wherein the selectivity of the step of selectively repeating (S128) comprises determining to repeat the steps (S116; S118; S120; S126) if the further distance metric, or any still further distance metric, exceeds a predetermined threshold; and/or wherein the selectivity of the step of selectively repeating (S128) the steps comprises determining to not repeat the steps (S116; S118; S120; S126) if the further distance metric, or any still further distance metric, falls below the predetermined threshold.

2. Method (100) according to claim 1, further comprising the steps of:

29 **EP 4 339 883 B1** 30

- Receiving (S122), at the UI (212), at least one correction by the user on the output (S120) third segmentation; and

- Determining (S124), based on the output (S120) third segmentation, the fourth segmentation taking into account the at least one received (S122) correction to the third segmentation.

3. Method (100) according to any one of the preceding claims, wherein the NN (202) comprises a convolutional neural network, CNN (202), optionally wherein the CNN (202) comprises a U-net and/or one or more residual neural network, ResNet, encoders.

4. Method (100) according to any one of the preceding claims, wherein the annotated segmentation (502-1; 502-2), the first segmentation (304; 304-1; 304-2), the second segmentation (312; 312-1; 312-2), the third segmentation, the fourth segmentation, and/or any further segmentation comprises a binary segmentation, wherein the binary segmentation comprises classifying each pixel of the medical image as background and/or as object, in particular as organ.

5. Method (100) according to any one of the preceding claims, wherein the annotated segmentation (502-1; 502-2), the first segmentation (304; 304-1; 304-2), the second segmentation (312; 312-1; 312-2), the third segmentation, the fourth segmentation, and/or any further segmentation comprises a K-ary segmentation with K equal to, or greater than, three, wherein the K-ary segmentation comprises classifying each pixel of the medical image as background, as foreground, and/or as organ,

wherein classifying a pixel as organ further comprises classifying a type of organ, and/or wherein classifying a pixel as foreground further comprises classifying a type of foreground.

6. Method (100) according to any one of the preceding claims, wherein the at least one correction by the user on the first segmentation (304; 304-1; 304-2), on the third segmentation, and/or on any further segmentation, comprises a manual correction performed by the user.

7. Method (100) according to any one of the preceding claims, wherein the at least one correction by the user on the first segmentation (304; 304-1; 304-2), on the third segmentation, and/or on any further segmentation, comprises a simulated correction.

8. Method (100) according to any one of the preceding claims, wherein the at least one correction by the user is selected from the group, consisting of:

- at least a piece of a contour, in particular of an object;
- at least a piece of a border, in particular between objects;
- a selection as interior of an object;
- a selection as exterior of an object; and
- a marking of a position.

9. Method (100) according to any one of the preceding claims, wherein the at least one correction by the user on the first segmentation (304; 304-1; 304-2), on the third segmentation, and/or on any further segmentation, is performed on a localized region of interest, ROI, optionally wherein the second segmentation (312; 312-1; 312-2), the fourth segmentation, and/or any further segmentation based on the at least one correction by the user only differs from the first segmentation (304; 304-1; 304-2), the third segmentation, and/the preceding further segmentation output by the NN (202), respectively, only within, or near, the ROI.

10. Method (100) according to any one of the preceding claims, wherein the medical image comprises a two-dimensional image, and/or wherein the medical image comprises a three-dimensional image, in particular a stack of slices.

11. Method (100) according to any one of the preceding claims, wherein the medical image is acquired from a medical imaging device using a medical image modality, which is selected from the group, consisting of:

- magnetic resonance imaging, MRI;
- computed tomography, CT;
- Ultrasound, US;
- positron emission tomography, PET;
- single-photon emission computed tomography, SPECT;
- angiography; and
- radiography.

12. Method (100) according to any one of the preceding claims, wherein the distance metric (310) comprises a Euclidean metric and/or a geodesic metric, optionally wherein the distance metric (310) comprises a, in particular weighted, sum of the Euclidean metric and the geodesic metric.

13. Method (100) according to any one of the preceding claims, wherein training the NN (202) comprises applying any one of the preceding method steps to a plurality, or all, first medical image datasets comprised in the training database.

14. Method (100) according to any one of the preceding claims, further comprising an inference phase, wherein the inference phase comprises:

- receiving, at the input layer (204) of the NN (202), a further medical image from a medical imaging device;
- performing, by the one or more hidden layers (206) of the NN (202) with interconnections (208), a segmentation of anatomical structures comprised in the received further medical image, wherein the interconnections (208) have been determined in the training phase; and
- outputting the segmentation of the anatomical structures of the further medical image.

15. Neural network system (200) for training a neural network, NN (202), for interactive medical image segmentation, the system comprising:

- the NN (202) with:

  ∘ an input layer (204) configured to receive a first medical image dataset from a training database of medical image datasets as input to the NN (202), wherein the first medical image dataset comprises a medical image (302) and optionally an annotated segmentation (502-1; 502-2);
  ∘ one or more hidden layers (206) with interconnections (208) configured to perform a first segmentation (304; 304-1; 304-2) of anatomic structures comprised in the medical image (302) of the received first medical image dataset;
  o an output layer (210) configured to output the performed first segmentation (304; 304-1; 304-2) for corrections (306) by a user;

- a user interface, UI (212), configured to receive at least one correction (306) by the user on the output first segmentation (304; 304-1; 304-2);
- a determining unit (216) configured to determine, based on the output first segmentation (304; 304-1; 304-2), a second segmentation (312; 312-1; 312-2) taking into account the at least one received correction (306) to the first segmentation (304; 304-1; 304-2);
- an annotation reception interface (214) configured to receive the annotated segmentation (502-1; 502-2) of the first medical image dataset;
- a computing unit (218) configured to determine a distance metric (310) between the determined second segmentation (312; 312-1; 312-2) and the annotated segmentation (502-1; 502-2) comprised in the received medical image dataset in association with the medical image (302) and/or between the determined second segmentation (312; 312-1; 312-2) and the output first segmentation (304; 304-1; 304-2); and
- a combining unit (220) configured to combine the determined distance metric (310), the determined second segmentation (312; 312-1; 312-2) and the medical image (302) into a second medical image dataset (314);

  wherein the input layer (204) of the NN (202) is further configured to receive the combined second medical image dataset (314) as input to the NN (202);
  wherein the one or more hidden layers (206) of the NN (202) with interconnections (208) are further configured to perform a third segmentation of the anatomic structures comprised in the medical image of the received second medical image dataset (314), wherein performing the third segmentation comprises changing one or more weights of the interconnections (208) of the NN (202), wherein the changing of the one or more weights of the interconnections (208) of the NN (202) is based on the determined distance metric (310) comprised in the second medical image dataset (314);
  wherein the output layer (210) of the NN (202) is further configured to output the performed third segmentation;
  wherein the computing unit is further configured to determine a further distance metric between the output third segmentation, and/or a fourth segmentation based on the third segmentation, and the annotated segmentation (502-1; 502-2) comprised in the received second medical image dataset (314) and/or between the output third segmentation, and/or the fourth segmentation based on the third segmentation, and the determined second segmentation, and/or the output third segmentation; and
  wherein the NN (202) is further configured to selectively repeat at least the steps of receiving a combined further medical image data set, performing a further segmentation of the anatomic structures comprised in the medical image (302) of the received further medical image data set, outputting the performed further segmentation, and determining a still further distance metric between the output further segmentation and the annotated segmentation (502-1; 502-2) and/or between the output further segmentation and the directly preceding segmentation;
  wherein the selectivity of the step of selectively repeating comprises determining to repeat the steps if the further distance metric, or any still further distance metric, exceeds a predetermined threshold; and
  wherein the selectivity of the step of selectively repeating comprises determining to not repeat the steps if the further distance metric, or any still further distance metric, falls below the predetermined threshold.

16. Neural network system (200) according to the directly preceding claim, further configured to perform any one of the steps, or comprise any one of the features, of the method claims 2 to 14.

17. A computer program product comprising program elements which induce a neural network system (200) to carry out the steps of the method for training a neural network, NN (202), for interactive medical image segmentation according to any one of the preceding method claims, when the program elements are loaded into a memory of the neural network system (200).

18. A computer-readable medium on which program elements are stored that can be read and executed by a neural network system (200), in order to perform steps of the method for training a neural network, NN (202), for interactive medical image segmentation according to any one of the preceding method claims, when the program elements are executed by the neural network system (200).

**Patentansprüche**

1. Computerimplementiertes Verfahren (100) zum Trainieren eines neuronalen Netzwerks, NN (202), für interaktive medizinische Bildesegmentierung, wobei eine Trainingsphase des NNs (202) die folgenden Verfahrensschritte umfasst:

- Empfangen (S102) eines ersten medizinischen Bilddatensatzes von einer Trainingsdatenbank von medizinischen Bilddatensätzen als Eingabe in das NN (202) an einer Eingabeschicht (204) des NNs (202), wobei der erste medizinische Bilddatensatz ein medizinisches Bild (302) und gegebenenfalls eine annotierte Segmentierung (502-1; 502-2) umfasst;
- Durchführen (S104) einer ersten Segmentierung (304; 304-1; 304-2) anatomischer Strukturen, die in dem medizinischen Bild (302) des empfangenen (S102) ersten medizinischen Bilddatensatzes enthalten sind, durch eine oder mehrere verborgene Schichten (206) des NNs (202) mit Verknüpfungen (208);
- Ausgeben (S106) der durchgeführten (S104) ersten Segmentierung (304; 304-1; 304-2) an einer Ausgabeschicht (210) des NNs (202) für Korrekturen (306) durch einen Benutzer;
- Empfangen (S108) mindestens einer Korrektur (306) durch den Benutzer an der ausgegebenen (S106) ersten Segmentierung (304; 304-1; 304-2) an einer Benutzerschnittstelle, UI (212);
- Bestimmen (S110) einer zweiten Segmentierung (312; 312-1; 312-2) unter Berücksichtigung der mindestens einen empfangenen (S108) Korrektur (306) der ersten Segmentierung (304; 304-1; 304-2) basierend auf der ausgegebenen (S106) ersten Segmentierung (304; 304-1; 304-2);
- Bestimmen (S112) einer Abstandsmetrik (310) zwischen der bestimmten (S110) zweiten Segmentierung (312; 312-1; 312-2) und der annotierten Segmentierung (502-1; 502-2), die in dem empfangenen (S102) medizinischen Bilddatensatz in Assoziation mit dem medizinischen Bild (302) enthalten ist, und/oder zwischen der bestimmten (S110) zweiten Segmentierung (312; 312-1; 312-2) und der ausgegebenen (S106) ersten Segmentierung (304; 304-1; 304-2);
- Kombinieren (S114) der bestimmten (S112) Abstandsmetrik (310), der bestimmten (S110) zweiten Segmentierung (312; 312-1; 312-2) und des medizinischen Bildes (302) zu einem zweiten medizinischen Bilddatensatz (314);
- Empfangen (S116) des kombinierten (S114) zweiten medizinischen Bilddatensatzes (314) als Eingabe in das NN (202) an der Eingabeschicht (204) des NNs (202);
- Durchführen (S118) einer dritten Segmentierung der anatomischen Strukturen, die in dem medizinischen Bild des empfangenen (S116) zweiten medizinischen Bilddatensatzes (314) enthalten sind, durch die eine oder die mehreren verborgenen Schichten (206) des NNs (202) mit Verknüpfungen (208), wobei das Durchführen (S118) der dritten Segmentierung ein Ändern einer oder mehrerer Gewichtungen der Verknüpfungen (208) des NNs (202) umfasst, wobei das Ändern der einen oder mehreren Gewichtungen der Verknüpfungen (208) des NNs (202) auf der bestimmten (S112) Abstandsmetrik (310) basiert, die in dem zweiten medizinischen Bilddatensatz (314) enthalten ist;
- Ausgeben (S120) der durchgeführten (S118) dritten Segmentierung an der Ausgabeschicht (210) des NNs (202);
- Bestimmen (S126) einer weiteren Abstandsmetrik zwischen der ausgegebenen (S120) dritten Segmentierung und/oder einer vierten Segmentierung basierend auf der dritten Segmentierung und der annotierten Segmentierung (502-1; 502-2), die in dem empfangenen (S116) zweiten medizinischen Bilddatensatz (314) enthalten ist, und/oder zwischen der ausgegebenen (S120) dritten Segmentierung und/oder der vierten Segmentierung basierend auf der dritten Segmentierung und der bestimmten (S110) zweiten Segmentierung (312; 312-1; 312-2) und/oder der ausgegebenen (S120) dritten Segmentierung; und

- selektives Wiederholen (S128) mindestens der Schritte des Empfangens (S116) eines kombinierten weiteren medizinischen Bilddatensatzes, des Durchführens (S118) einer weiteren Segmentierung der anatomischen Strukturen, die in dem medizinischen Bild (302) des empfangenen (S116) weiteren medizinischen Bilddatensatzes enthalten sind, des Ausgebens (S120) der durchgeführten weiteren Segmentierung und des Bestimmens (S126) noch einer weiteren Abstandsmetrik zwischen der ausgegebenen (S120) weiteren Segmentierung und der annotierten Segmentierung (502-1; 502-2) und/oder zwischen der ausgegebenen (S120) weiteren Segmentierung und der direkt vorhergehenden Segmentierung;

wobei die Selektivität des Schritts des selektiven Wiederholens (S128) ein Bestimmen umfasst, die Schritte (S116; S118; S120; S126) zu wiederholen, falls die weitere Abstandsmetrik oder eine beliebige noch weitere Abstandsmetrik eine vorbestimmte Schwelle überschreitet; und/oder wobei die Selektivität des Schritts des selektiven Wiederholens (S128) der Schritte ein Bestimmen umfasst, die Schritte (S116; S118; S120; S126) nicht zu wiederholen, falls die weitere Abstandsmetrik oder eine beliebige noch weitere Abstandsmetrik unter die vorbestimmte Schwelle fällt.

2. Verfahren (100) nach Anspruch 1, ferner umfassend die Schritte:

- Empfangen (S122) mindestens einer Korrektur durch den Benutzer an der ausgegebenen (S120) dritten Segmentierung an der UI (212); und
- Bestimmen (S124) der vierten Segmentierung unter Berücksichtigung der mindestens einen empfangenen (S122) Korrektur der dritten Segmentierung basierend auf der ausgegebenen (S120) dritten Segmentierung.

3. Verfahren (100) nach einem der vorhergehenden Ansprüche, wobei das NN (202) ein neuronales Faltungsnetzwerk, CNN (202), umfasst, wobei das CNN (202) gegebenenfalls ein U-Net und/oder einen oder mehrere ResNet-Encoder (ResNet = neuronales Residualnetzwerk) umfasst.

4. Verfahren (100) nach einem der vorhergehenden Ansprüche, wobei die annotierte Segmentierung (502-1; 502-2), die erste Segmentierung (304;

304-1; 304-2), die zweite Segmentierung (312; 312-1; 312-2), die dritte Segmentierung, die vierte Segmentierung, und/oder eine beliebige weitere Segmentierung eine binäre Segmentierung umfasst, wobei die binäre Segmentierung ein Klassifizieren jedes Pixels des medizinischen Bildes als Hintergrund und/oder als Objekt, insbesondere als Organ, umfasst.

5. Verfahren (100) nach einem der vorhergehenden Ansprüche, wobei die annotierte Segmentierung (502-1; 502-2), die erste Segmentierung (304; 304-1; 304-2), die zweite Segmentierung (312; 312-1; 312-2), die dritte Segmentierung, die vierte Segmentierung, und/oder eine beliebige weitere Segmentierung eine K-äre Segmentierung mit K gleich oder größer als drei umfasst, wobei die K-äre Segmentierung ein Klassifizieren jedes Pixels des medizinischen Bildes als Hintergrund, als Vordergrund und/oder als Organ umfasst, wobei das Klassifizieren eines Pixels als Organ ferner ein Klassifizieren eines Organtyps umfasst und/oder wobei ein Klassifizieren eines Pixels als Vordergrund ferner ein Klassifizieren eines Vordergrundtyps umfasst.

6. Verfahren (100) nach einem der vorhergehenden Ansprüche, wobei die mindestens eine Korrektur durch den Benutzer an der ersten Segmentierung (304; 304-1; 304-2), an der dritten Segmentierung und/oder an einer beliebigen weiteren Segmentierung eine manuelle Korrektur umfasst, die durch den Benutzer durchgeführt wird.

7. Verfahren (100) nach einem der vorhergehenden Ansprüche, wobei die mindestens eine Korrektur durch den Benutzer an der ersten Segmentierung (304; 304-1; 304-2), an der dritten Segmentierung und/oder an einer beliebigen weiteren Segmentierung eine simulierte Korrektur umfasst.

8. Verfahren (100) nach einem der vorhergehenden Ansprüche, wobei die mindestens eine Korrektur durch den Benutzer aus der Gruppe ausgewählt wird, die aus Folgendem besteht:

- mindestens einem Teil einer Kontur, insbesondere eines Objekts;
- mindestens einem Teil einer Grenze, insbesondere zwischen Objekten;
- einer Auswahl als Inneres eines Objekts;
- einer Auswahl als Äußeres eines Objekts; und
- einer Markierung einer Position.

9. Verfahren (100) nach einem der vorhergehenden Ansprüche, wobei die mindestens eine Korrektur durch den Benutzer an der ersten Segmentierung (304; 304-1; 304-2), an der dritten Segmentierung und/oder an einer beliebigen weiteren Segmentierung an einer lokalisierten Interessensregion, ROI, durchgeführt wird, wobei gegebenenfalls die zweite Segmentierung (312; 312-1; 312-2), die vierte Segmentierung und/oder eine beliebige weitere Segmentierung basierend auf der mindestens einen Korrektur durch den Benutzer sich nur jeweils innerhalb oder nahe der ROI von der ersten Segmentierung (304; 304-1; 304-2), der dritten Segmentierung und/oder der vorhergehenden weiteren Segmentierung unterscheidet, die durch das NN (202) ausgegeben wird.

10. Verfahren (100) nach einem der vorhergehenden Ansprüche, wobei das medizinische Bild ein zweidimensionales Bild umfasst und/oder wobei das medizinische Bild ein dreidimensionales Bild, insbesondere einen Stapel von Schichten, umfasst.

11. Verfahren (100) nach einem der vorhergehenden Ansprüche, wobei das medizinische Bild von einer medizinischen Bildgebungsvorrichtung unter Verwendung einer medizinischen Bildmodalität erfasst wird, die aus der Gruppe ausgewählt ist, die aus Folgendem besteht:

   - Magnetresonanztomographie, MRT;
   - Computertomographie, CT;
   - Ultraschall, US;
   - Positronen-Emissionstomographie, PET;
   - Einzelphotonenemission-Computertomographie, SPECT;
   - Angiographie; und
   - Radiographie.

12. Verfahren (100) nach einem der vorhergehenden Ansprüche, wobei die Abstandsmetrik (310) eine euklidische Metrik und/oder eine geodätische Metrik umfasst, wobei die Abstandsmetrik (310) gegebenenfalls eine, insbesondere gewichtete, Summe der euklidischen Metrik und der geodätischen Metrik umfasst.

13. Verfahren (100) nach einem der vorhergehenden Ansprüche, wobei Trainieren des NNs (202) ein Anwenden eines beliebigen der vorhergehenden Verfahrensschritte auf eine Vielzahl von oder alle ersten medizinischen Bilddatensätze umfasst, die in der Trainingsdatenbank enthalten sind.

14. Verfahren (100) nach einem der vorhergehenden Ansprüche, ferner umfassend eine Inferenzphase, wobei die Inferenzphase umfasst:

   - Empfangen eines weiteren medizinischen Bildes von einer medizinischen Bildgebungsvorrichtung an der Eingabeschicht (204) des NNs (202);

   - Durchführen einer Segmentierung anatomischer Strukturen, die in dem empfangenen weiteren medizinischen Bild enthalten sind, durch die eine oder mehreren verborgenen Schichten (206) des NNs (202) mit Verknüpfungen (208), wobei die Verknüpfungen (208) in der Trainingsphase bestimmt wurden; und
   - Ausgeben der Segmentierung der anatomischen Strukturen des weiteren medizinischen Bildes.

15. Neuronales Netzwerksystem (200) zum Trainieren eines neuronalen Netzwerks, NN (202), für eine interaktive medizinische Bildsegmentierung, wobei das System umfasst:

   - das NN (202) mit:

      o einer Eingabeschicht (204), die dazu ausgelegt ist, einen ersten medizinischen Bilddatensatz von einer Trainingsdatenbank von medizinischen Bilddatensätzen als Eingabe in das NN (202) zu empfangen, wobei der erste medizinische Bilddatensatz ein medizinisches Bild (302) und gegebenenfalls eine annotierte Segmentierung (502-1; 502-2) umfasst;
      o einer oder mehreren verborgenen Schichten (206) mit Verknüpfungen (208), die dazu ausgelegt ist/sind, eine erste Segmentierung (304; 304-1; 304-2) anatomischer Strukturen durchzuführen, die in dem medizinischen Bild (302) des empfangenen ersten medizinischen Bilddatensatzes enthalten sind;
      o einer Ausgabeschicht (210), die dazu ausgelegt ist, die durchgeführte erste Segmentierung (304; 304-1; 304-2) für Korrekturen (306) durch einen Benutzer auszugeben;

   - eine Benutzerschnittstelle, UI (212), die dazu ausgelegt ist, mindestens eine Korrektur (306) durch den Benutzer an der ausgegebenen ersten Segmentierung (304; 304-1; 304-2) zu empfangen;
   - eine Bestimmungseinheit (216), die dazu ausgelegt ist, basierend auf der ausgegebenen ersten Segmentierung (304; 304-1; 304-2) eine zweite Segmentierung (312; 312-1; 312-2) unter Berücksichtigung der mindestens einen empfangenen Korrektur (306) an der ersten Segmentierung (304; 304-1; 304-2) zu bestimmen;
   - eine Annotationsempfangsschnittstelle (214), die dazu ausgelegt ist, die annotierte Segmentierung (502-1; 502-2) des ersten medizinischen Bilddatensatzes zu empfangen;
   - eine Recheneinheit (218), die dazu ausgelegt ist, eine Abstandsmetrik (310) zwischen der bestimmten zweiten Segmentierung (312; 312-1; 312-2) und der in dem empfangenen medizinischen Bilddatensatz enthaltenen annotierten Segmentierung (502-1; 502-2) in Assoziation mit dem medizinischen Bild (302) und/oder zwischen der bestimmten zweiten Segmentierung (312; 312-1; 312-2) und der ausgegebenen ersten Segmentierung (304; 304-1; 304-2) zu bestimmen; und

- eine Kombinationseinheit (220), die dazu ausgelegt ist, die bestimmte Abstandsmetrik (310), die bestimmte zweite Segmentierung (312; 312-1; 312-2) und das medizinische Bild (302) zu einem zweiten medizinischen Bilddatensatz (314) zu kombinieren;

wobei die Eingabeschicht (204) des NNs (202) ferner dazu ausgelegt ist, den kombinierten zweiten medizinischen Bilddatensatz (314) als Eingabe in das NN (202) zu empfangen;

wobei die eine oder mehreren verborgenen Schichten (206) des NNs (202) mit Verknüpfungen (208) ferner dazu ausgelegt ist/sind, eine dritte Segmentierung der anatomischen Strukturen durchzuführen, die in dem medizinischen Bild des empfangenen zweiten medizinischen Bilddatensatzes (314) enthalten sind, wobei das Durchführen der dritten Segmentierung ein Ändern einer oder mehrerer Gewichtungen der Verknüpfungen (208) des NNs (202) umfasst, wobei das Ändern der einen oder mehreren Gewichtungen der Verknüpfungen (208) des NNs (202) auf der bestimmten Abstandsmetrik (310) basiert, die in dem zweiten medizinischen Bilddatensatz (314) enthalten ist;

wobei die Ausgabeschicht (210) des NNs (202) ferner dazu ausgelegt ist, die durchgeführte dritte Segmentierung auszugeben;

wobei die Recheneinheit ferner dazu ausgelegt ist, eine weitere Abstandsmetrik zwischen der ausgegebenen dritten Segmentierung und/oder einer vierten Segmentierung basierend auf der dritten Segmentierung und der annotierten Segmentierung (502-1; 502-2), die in dem empfangenen zweiten medizinischen Bilddatensatz (314) enthalten ist, und/oder zwischen der ausgegebenen dritten Segmentierung und/oder der vierten Segmentierung basierend auf der dritten Segmentierung und der bestimmten zweiten Segmentierung und/oder der ausgegebenen dritten Segmentierung zu bestimmen; und

wobei das NN (202) ferner dazu ausgelegt ist, mindestens die Schritte des Empfangens eines kombinierten weiteren medizinischen Bilddatensatzes, des Durchführens einer weiteren Segmentierung der anatomischen Strukturen, die in dem medizinischen Bild (302) des empfangenen weiteren medizinischen Bilddatensatzes enthalten sind, des Ausgebens der durchgeführten weiteren Segmentierung und des Bestimmens noch einer weiteren Abstandsmetrik zwischen der ausgegebenen weiteren Segmentierung und der annotierten Segmentierung (502-1; 502-2) und/oder zwischen der ausgegebenen weiteren Segmentierung und der direkt vorhergehenden weiteren Segmentierung selektiv zu wiederholen;

wobei die Selektivität des Schritts des selektiven Wiederholens ein Bestimmen umfasst, die Schritte zu wiederholen, falls die weitere Abstandsmetrik oder eine beliebige noch weitere Abstandsmetrik eine vorbestimmte Schwelle überschreitet; und

wobei die Selektivität des Schritts des selektiven Wiederholens ein Bestimmen umfasst, die Schritte nicht zu wiederholen, falls die weitere Abstandsmetrik oder eine beliebige noch weitere Abstandsmetrik unter die vorbestimmte Schwelle fällt.

16. Neuronales Netzwerksystem (200) nach dem direkt vorhergehenden Anspruch, das ferner dazu ausgelegt ist, einen der Schritte durchzuführen oder eines der Merkmale der Verfahrensansprüche 2 bis 14 zu umfassen.

17. Computerprogrammprodukt, umfassend Programmelemente, die ein neuronales Netzwerksystem (200) veranlassen, die Schritte des Verfahrens zum Trainieren eines neuronalen Netzwerks, NN (202), zur interaktiven medizinischen Bildsegmentierung nach einem der vorhergehenden Verfahrensansprüche auszuführen, wenn die Programmelemente in einen Speicher des neuronalen Netzwerksystems (200) geladen werden.

18. Computerlesbares Medium, auf dem Programmelemente gespeichert sind, die durch ein neuronales Netzwerksystem (200) gelesen und ausgeführt werden können, um Schritte des Verfahrens zum Trainieren eines neuronalen Netzwerks, NN (202), für interaktive medizinische Bildsegmentierung gemäß einem der vorhergehenden Verfahrensansprüche durchzuführen, wenn die Programmelemente durch das neuronale Netzwerksystem (200) ausgeführt werden.

## Revendications

1.  Procédé mis en œuvre par ordinateur (100) d'apprentissage d'un réseau neuronal, NN (202), pour la segmentation interactive d'images médicales, une phase d'apprentissage du NN (202) comprenant les étapes de procédé de :

    - réception (S102), au niveau d'une couche d'entrée (204) du NN (202), d'un premier ensemble de données d'images médicales provenant d'une base de données d'apprentissage d'ensembles de données d'images médicales comme entrée dans le NN (202), le premier ensemble de données d'images médicales comprenant une image médicale (302) et éventuellement une segmentation annotée (502-1 ; 502-2) ;
    - réalisation (S104), par une ou plusieurs couches cachées (206) du NN (202) avec des interconnexions (208), d'une première segmentation (304 ; 304-1 ; 304-2) de structures anatomiques comprises dans l'image médicale (302) du premier ensemble de données d'images médicales reçu (S102) ;
    - sortie (S106), au niveau d'une couche de sortie (210) du NN (202), de la première segmentation (304 ; 304-1 ; 304-2) réalisée (S104) pour des corrections (306) par un utilisateur ;
    - réception (S108), au niveau d'une interface utilisateur, UI (212), d'au moins une correction (306) par l'utilisateur sur la première segmentation (304 ; 304-1 ; 304-2) de sortie (S106) ;
    - détermination (S110), sur la base de la première segmentation (304 ; 304-1 ; 304-2) de sortie (S106), d'une deuxième segmentation (312 ; 312-1 ; 312-2) tenant compte de l'au moins une correction (306) reçue (S108) de la première segmentation (304 ; 304-1 ; 304-2) ;
    - détermination (S112) d'une métrique de distance (310) entre la deuxième segmentation (312 ; 312-1 ; 312-2) déterminée (S110) et la segmentation annotée (502-1 ; 502-2) comprise dans l'ensemble de données d'images médicales reçu (S102) en association avec l'image médicale (302) et/ou entre la deuxième segmentation (312 ; 312-1 ; 312-2) déterminée (S110) et la première segmentation (304 ; 304-1 ; 304-2) de sortie (S106) ;
    - combinaison (S114) de la métrique de distance (310) déterminée (S112), de la deuxième segmentation (312 ; 312-1 ; 312-2) déterminée (S110) et de l'image médicale (302) en un deuxième ensemble de données d'images médicales (314) ;
    - réception (S116), au niveau de la couche d'entrée (204) du NN (202), du deuxième ensemble de données d'images médicales (314) combiné (S114) en tant qu'entrée dans le NN (202) ;
    - réalisation (S118), par la ou les couches cachées (206) du NN (202) avec interconnexions (208), d'une troisième segmentation des structures anatomiques comprises dans l'image médicale du deuxième ensemble de données d'images médicales (314) reçu (S116), la réalisation (S118) de la troisième segmentation comprenant la modification d'un ou plusieurs poids des interconnexions (208) du NN (202), la modification du ou des poids des interconnexions (208) du NN (202) étant basée sur la métrique de distance (310) déterminée (S112) comprise dans le deuxième ensemble de données d'images médicales (314) ;
    - sortie (S120), au niveau de la couche de sortie (210) du NN (202), de la troisième segmentation réalisée (S118) ;
    - détermination (S126) d'une métrique de distance supplémentaire entre la troisième segmentation de sortie (S120) et/ou une quatrième segmentation basée sur la troisième segmentation, et la segmentation annotée (502-1 ; 502-2) comprise dans le deuxième ensemble de données d'images médicales (314) reçu (S116) et/ou entre la troisième segmentation de sortie (S120) et/ou la quatrième segmentation basée sur la troisième segmentation, et la deuxième segmentation (312 ; 312-1 ; 312-2) déterminée (S110) et/ou la troisième segmentation de sortie (S120) ; et
    - répétition sélective (S128) d'au moins les étapes de réception (S116) d'un ensemble combiné de données d'images médicales supplémentaire, réalisation (S118) d'une segmentation supplémentaire des structures anatomiques comprises dans l'image médicale (302) de l'ensemble de données d'images médicales encore supplémentaire reçu (S116), sortie (S120) de la segmentation supplémentaire réalisée, et détermination (S126) d'une métrique de distance encore supplémentaire entre la segmentation supplémentaire de sortie (S120) et la segmentation annotée (502-1 ; 502-2) et/ou entre la segmentation supplémentaire de sortie (S120) et la segmentation directement précédente ;

      la sélectivité de l'étape de répétition sélective (S128) comprenant la détermination de répéter les étapes (S116 ; S118 ; S120 ; S126) si la métrique de distance supplémentaire, ou toute métrique de distance encore supplémentaire, dépasse un seuil prédéterminé ; et/ou
      la sélectivité de l'étape de répétition sélective (S128) des étapes comprenant la détermination de ne pas répéter les étapes (S116 ; S118 ; S120 ; S126) si la métrique de distance supplémentaire, ou toute métrique de distance encore supplémentaire, tombe en dessous du seuil prédéterminé.

**2.** Procédé (100) selon la revendication 1, comprenant en outre les étapes de :

- réception (S122), au niveau de l'UI (212), d'au moins une correction par l'utilisateur sur la troisième segmentation de sortie (S120) ; et
- détermination (S124), sur la base de la troisième segmentation de sortie (S120), de la quatrième segmentation prenant en compte l'au moins une correction reçue (S122) de la troisième segmentation.

**3.** Procédé (100) selon l'une quelconque des revendications précédentes, le NN (202) comprenant un réseau neuronal convolutif, CNN (202), éventuellement le CNN (202) comprenant un réseau U et/ou un ou plusieurs codeurs de réseau neuronal résiduel, RESNET.

**4.** Procédé (100) selon l'une quelconque des revendications précédentes, la segmentation annotée (502-1 ; 502-2), la première segmentation (304 ; 304-1 ; 304-2), la deuxième segmentation (312 ; 312-1 ; 312-2), la troisième segmentation, la quatrième segmentation, et/ou toute autre segmentation comprenant une segmentation binaire, la segmentation binaire comprenant la classification de chaque pixel de l'image médicale comme arrière-plan et/ou comme objet, en particulier comme organe.

**5.** Procédé (100) selon l'une quelconque des revendications précédentes, la segmentation annotée (502-1 ; 502-2), la première segmentation (304 ; 304-1 ; 304-2), la deuxième segmentation (312 ; 312-1 ; 312-2), la troisième segmentation, la quatrième segmentation, et/ou toute autre segmentation comprenant une segmentation K-aire avec K égal ou supérieur à trois, la segmentation K-aire comprenant la classification de chaque pixel de l'image médicale en tant qu'arrière-plan, avant-plan et/ou organe, la classification d'un pixel en tant qu'organe comprenant en outre la classification d'un type d'organe, et/ou la classification d'un pixel en tant qu'avant-plan comprenant en outre la classification d'un type d'avant-plan.

**6.** Procédé (100) selon l'une quelconque des revendications précédentes, l'au moins une correction par l'utilisateur sur la première segmentation (304 ; 304-1 ; 304-2), sur la troisième segmentation, et/ou sur toute autre segmentation, comprenant une correction manuelle réalisée par l'utilisateur.

**7.** Procédé (100) selon l'une quelconque des revendications précédentes, l'au moins une correction par l'utilisateur sur la première segmentation (304 ; 304-1 ; 304-2), sur la troisième segmentation, et/ou sur toute autre segmentation, comprenant une correction simulée.

**8.** Procédé (100) selon l'une quelconque des revendications précédentes, l'au moins une correction par l'utilisateur étant sélectionnée dans le groupe, consistant en :

- au moins une partie d'un contour, en particulier d'un objet ;
- au moins une partie de bordure, notamment entre objets ;
- une sélection comme intérieur d'un objet ;
- une sélection comme extérieur d'un objet ; et
- un marquage d'une position.

**9.** Procédé (100) selon l'une quelconque des revendications précédentes, l'au moins une correction par l'utilisateur sur la première segmentation (304 ; 304-1 ; 304-2), sur la troisième segmentation, et/ou sur toute autre segmentation, étant réalisée sur une région d'intérêt localisée, ROI, éventuellement la deuxième segmentation (312 ; 312-1 ; 312-2), la quatrième segmentation, et/ou toute autre segmentation basée sur l'au moins une correction par l'utilisateur ne différant que de la première segmentation (304 ; 304-1 ; 304-2), la troisième segmentation et/ou la segmentation supplémentaire précédente délivrée par le NN (202), respectivement, uniquement à l'intérieur ou à proximité de la ROI.

**10.** Procédé (100) selon l'une quelconque des revendications précédentes, l'image médicale comprenant une image bidimensionnelle, et/ou l'image médicale comprenant une image tridimensionnelle, en particulier une pile de tranches.

**11.** Procédé (100) selon l'une quelconque des revendications précédentes, l'image médicale étant acquise à partir d'un dispositif d'imagerie médicale en utilisant une modalité d'image médicale, qui est sélectionnée dans le groupe, consistant en :

- l'imagerie par résonance magnétique, IRM ;
- la tomodensitométrie, CT ;
- l'échographie, US ;
- la tomographie par émission de positons, PET ;
- la tomographie informatisée d'émission monophotonique, SPECT ;
- l'angiographie ; et
- la radiographie.

**12.** Procédé (100) selon l'une quelconque des revendications précédentes, la métrique de distance (310) comprenant une métrique euclidienne et/ou une métrique géodésique, le cas échéant, la métrique de distance (310) comprenant une somme, en particulier pondérée, de la métrique euclidienne et de la métrique géodésique.

13. Procédé (100) selon l'une quelconque des revendications précédentes, l'apprentissage du NN (202) comprenant l'application de l'une quelconque des étapes de procédé précédentes à une pluralité, ou à la totalité, des premiers ensembles de données d'images médicales compris dans la base de données d'apprentissage.

14. Procédé (100) selon l'une quelconque des revendications précédentes, comprenant en outre une phase d'inférence, la phase d'inférence comprenant :

- la réception, au niveau de la couche d'entrée (204) du NN (202), d'une autre image médicale provenant d'un dispositif d'imagerie médicale ;
- la réalisation, par la ou les couches cachées (206) du NN (202) avec interconnexions (208), d'une segmentation des structures anatomiques comprises dans l'image médicale supplémentaire reçue, les interconnexions (208) ayant été déterminées dans la phase d'apprentissage ; et
- la sortie de la segmentation des structures anatomiques de l'image médicale supplémentaire.

15. Système de réseau neuronal (200) pour l'apprentissage d'un réseau neuronal, NN (202), pour la segmentation interactive d'images médicales, le système comprenant :

- le NN (202) avec :

o une couche d'entrée (204) configurée pour recevoir un premier ensemble de données d'images médicales provenant d'une base de données d'apprentissage d'ensembles de données d'images médicales en tant qu'entrée dans le NN (202), le premier ensemble de données d'images médicales comprenant une image médicale (302) et éventuellement une segmentation annotée (502-1 ; 502-2) ;
o une ou plusieurs couches cachées (206) avec des interconnexions (208) configurées pour réaliser une première segmentation (304 ; 304-1 ; 304-2) de structures anatomiques comprises dans l'image médicale (302) du premier ensemble de données d'images médicales reçu ;
o une couche de sortie (210) configurée pour sortir la première segmentation réalisée (304 ; 304-1 ; 304-2) pour des corrections (306) par un utilisateur ;

- une interface utilisateur, UI (212), configurée pour recevoir au moins une correction (306) par l'utilisateur sur la première segmentation de sortie (304 ; 304-1 ; 304-2) ;
- une unité de détermination (216) configurée pour déterminer, sur la base de la première segmentation de sortie (304 ; 304-1 ; 304-2), une deuxième segmentation (312 ; 312-1 ; 312-2) tenant compte de l'au moins une correction reçue (306) de la première segmentation (304 ; 304-1 ; 304-2) ;
- une interface de réception d'annotation (214) configurée pour recevoir la segmentation annotée (502-1 ; 502-2) du premier ensemble de données d'images médicales ;
- une unité de calcul (218) configurée pour déterminer une métrique de distance (310) entre la deuxième segmentation déterminée (312 ; 312-1 ; 312-2) et la segmentation annotée (502-1 ; 502-2) comprise dans l'ensemble de données d'images médicales reçues en association avec l'image médicale (302) et/ou entre la deuxième segmentation déterminée (312 ; 312-1 ; 312-2) et la première segmentation de sortie (304 ; 304-1 ; 304-2) ; et
- une unité de combinaison (220) configurée pour combiner la métrique de distance déterminée (310), la deuxième segmentation déterminée (312 ; 312-1 ; 312-2) et l'image médicale (302) en un deuxième ensemble de données d'images médicales (314) ;

la couche d'entrée (204) du NN (202) étant en outre configurée pour recevoir le deuxième ensemble de données d'images médicales combiné (314) en tant qu'entrée dans le NN (202) ;
la ou les couches cachées (206) du NN (202) avec interconnexions (208) étant en outre configurées pour réaliser une troisième segmentation des structures anatomiques comprises dans l'image médicale du deuxième ensemble de données d'images médicales reçu (314), la réalisation de la troisième segmentation comprenant la modification d'une ou plusieurs pondérations des interconnexions (208) du NN (202), la modification du ou des poids des interconnexions (208) du NN (202) étant basée sur la métrique de distance déterminée (310) comprise dans le deuxième ensemble de données d'images médicales (314) ; la couche de sortie (210) du NN (202) étant en outre configurée pour sortir la troisième segmentation réalisée ; l'unité de calcul étant en outre configurée pour déterminer une métrique de distance supplémentaire entre la troisième segmentation de sortie et/ou une quatrième segmentation basée sur la troisième segmentation, et la segmentation annotée (502-1 ; 502-2) comprise dans le deuxième ensemble de données d'images médicales reçu (314) et/ou entre la troisième segmentation de sortie et/ou la quatrième segmentation basée sur la troisième segmentation, et la deuxième segmentation déterminée, et/ou la troisième segmentation de sortie ; et le NN (202) étant en outre configuré pour répéter sélectivement au moins les étapes consistant à recevoir un autre ensemble combiné de données d'images médicales, à réaliser une segmentation supplémentaire des structures anatomiques comprises dans l'image médicale (302) de l'autre ensemble reçu de données d'images médicales, à sortir l'autre segmentation réalisée et à déterminer une métrique de distance encore supplémentaire entre l'autre segmentation de sortie et la segmentation annotée (502-1 ; 502-2) et/ou entre la segmentation supplémentaire de sortie et la segmentation directement précédente ; la sélectivité de l'étape de répétition sélective comprenant la détermination de répéter les étapes si la métrique de distance supplémentaire, ou toute métrique de distance encore supplémentaire, dépasse un seuil prédéterminé ; et la sélectivité de l'étape de répétition sélective comprenant la détermination de ne pas répéter les étapes si la métrique de distance supplémentaire, ou toute métrique de distance encore supplémentaire, tombe en dessous du seuil prédéterminé.

16. Système de réseau neuronal (200) selon la revendication précédente, configuré en outre pour exécuter l'une quelconque des étapes, ou pour comprendre l'une quelconque des caractéristiques, selon les revendications de procédé 2 à 14.

17. Produit de programme informatique comprenant des éléments de programme qui induisent un système de réseau neuronal (200) à exécuter les étapes du procédé d'apprentissage d'un réseau neuronal, NN (202), pour la segmentation interactive d'images médicales selon l'une quelconque des revendications de procédé précédentes, lorsque les éléments de programme sont chargés dans une mémoire du système de réseau neuronal (200).

18. Support lisible par ordinateur sur lequel sont stockés des éléments de programme qui peuvent être lus et exécutés par un système de réseau neuronal (200), afin d'exécuter des étapes du procédé d'apprentissage d'un réseau neuronal, NN (202), pour la segmentation interactive d'images médicales selon l'une quelconque des revendications de procédé précédentes, lorsque les éléments de programme sont exécutés par le système de réseau neuronal (200).

# FIG 1

100

```
S102  ──────►  S116
  │              │
  ▼              ▼
S104           S118
  │              │
  ▼              ▼
S106           S120
  │              │
  ▼              ▼
S108           S122
  │              │
  ▼              ▼
S110           S124
  │              │
  ▼              ▼
S112           S126
  │              │
  ▼              ▼
S114 ─────────►S128
```

FIG 2

# FIG 3

EP 4 339 883 B1

# FIG 4

Initial Segmantation     User Interaction     Final Result

304-1     304-2     306     312-1     312-2

EP 4 339 883 B1

# FIG 5

Groundtruth       Initial Annotation + User Interaction       Refined Annotation

502-1      502-2      304-1      304-2     306     308     312-1      312-2

EP 4 339 883 B1

# FIG 6

502-1

Groundtruth

502-2

304-1

Initial Segmentation +
User Interaction

304-2    306

312-1

Final Result

312-2

EP 4 339 883 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020085382 A1
- JP 2022535219 A
- EP 3639240 B1

**Non-patent literature cited in the description**

- **RAJCHL, M** ; **LEE, M. C** ; **OKTAY, O** ; **KAMNITSAS, K.** ; **PASSERAT-PALMBACH, J** ; **BAI, W** ; **RUECKERT, D**. Deepcut: Object segmentation from bounding box annotations using convolutional neural networks.. *IEEE transactions on medical imaging*, 2016, vol. 36 (2), 674-683
- Geos: Geodesic image segmentation.. **CRIMINISI, A** ; **SHARP, T** ; **BLAKE, A**. In European Conference on Computer Vision. Springer, October 2008, 99-112
- **ROTHER, C.** ; **KOLMOGOROV, V** ; **BLAKE, A.** GrabCut'' interactive foreground extraction using iterated graph cuts.. *ACM transactions on graphics (TOG)*, 2004, vol. 23 (3), 309-314
- 4D active cut: An interactive tool for pathological anatomy modeling.. **WANG, B** ; **LIU, K. W** ; **PRASTAWA, K. M** ; **IRIMA, A.** ; **VESPA, P. M.** ; **VAN HORN, J. D** ; **GERIG, G**. 2014 IEEE 11th International Symposium on Biomedical Imaging (ISBI). IEEE., April 2014, 529-532
- **GIRSHICK, R.** ; **DONAHUE, J.** ; **DARRELL, T** ; **MALIK, J.** Rich feature hierarchies for accurate object detection and semantic segmentation. *Proceedings of the IEEE conference on computer vision and pattern recognition*, 2014, 580-587